# EUROPEAN PATENT SPECIFICATION

(11) **EP 1 179 073 B1**
(45) Date of publication and mention of the grant of the patent: **17.01.2007**
(21) Application number: 00956164.8
(22) Date of filing: 05.07.2000
(51) Int. Cl.: C12N 15/53, C12N 15/77, C12P 13/08

(54) **PROCESS FOR THE FERMENTATIVE PREPARATION OF L-AMINO ACIDS WITH AMPLIFICATION OF THE ZWF GENE**
VERFAHREN ZUR FERMENTATIVEN HERSTELLUNG VON L-AMINOSÄUREN DURCH VERSTÄRKUNG DES ZWF-GENS
PROCEDE POUR LA PREPARATION FERMENTATIVE D'ACIDES AMINES L AVEC AMPLIFICATION DU GENE ZWF

(30) Priority: 20.03.2000 US 531269
(43) Date of publication of application: 13.02.2002
(73) Proprietor: Degussa AG, 40474 Düsseldorf (DE); Forschungszentrum Jülich GmbH, 52425 Jülich (DE); National University of Ireland, Galway (IE)
(72) Inventor: BURKE, Kevin, Newcastle, Galway, County Galway (IE); SAHM, Hermann, 52428 Jülich (DE); EGGELING, Lothar, 52428 Jülich (DE); MORITZ, Bernd, 44801 Bochum (DE); DUNICAN, L., K., deceased (IE); MCCORMACK, Ashling, Athlone, County Westmeath (IE); STAPELTON, Cliona, Roscrea, County Tipperary (IE); MÖCKEL, Bettina, 40597 Düsseldorf (DE); THIERBACH, Georg, 33613 Bielefeld (DE)
(86) International application number: PCT/EP2000/006303
(87) International publication number: WO 2001/070995

(56) References cited:
- EP-A- 1 108 790
- WO-A2-01/00843
- JP-A- 9 224 661
- L. EGGELING ET AL.: "L-Glutamate and L-Lysine: traditional products with impetuous developments" APPLIED MICROBIOLOGY AND BIOTECHNOLOGY, vol. 52, August 1999 (1999-08), pages 146-153, XP000979507

## Description

The invention relates to a process for the fermentative preparation of L-lysine, and L-threonine, using coryneform bacteria in which at least the zwf gene is amplified.

### Prior art

L-Amino acids are used in animal nutrition, in human medicine and in the pharmaceuticals industry.

It is known that amino acids are prepared by fermentation of strains of coryneform bacteria, in particular Corynebacterium glutamicum. Because of its great importance, work is constantly being undertaken to improve the preparation process. Improvements to the process can relate to fermentation measures, such as e. g. stirring and supply of oxygen, or the composition of the nutrient media, such as e. g. the sugar concentration during the fermentation, or the working up to the product form by e. g. ion exchange chromatography, or the intrinsic output properties of the microorganism itself.

Methods of mutagenesis, selection and mutant selection are used to improve the output properties of these microorganisms. Strains which are resistant to antimetabolites, such as e. g. the threonine analogue α-amino-β-hydroxyvaleric acid (AHV), or are auxotrophic for metabolites of regulatory importance and produce L-amino acids such as e. g. threonine are obtained in this manner.

Methods of the recombinant DNA technique have also been employed for some years for improving the strain of Corynebacterium glutamicum strains which produce L-amino acids.

JP-A-9224661 (Mitsubishi Chem. Corp.) discloses a plasmid for the cloning of the zwf gene.

### Object of the invention

The inventors had the object of providing new improved processes for the fermentative preparation of L-lysine and L-threonine with coryneform bacteria.

### Description of the invention

L-Amino acids are used in human medicine and in the pharmaceuticals industry, in the foodstuffs industry and especially in animal nutrition. There is therefore a general interest in providing new improved processes for the preparation of amino acids.

The invention provides an isolated gene of Corynebacterium glutamicum (zwf gene) obtainable by PCR from C. glutamicum ATCC BO32 from C. glutamicum ATCC13032 by means of the oligonucleotide primer
zwf-forward (SEQ ID NO:10):
5'-TCG ACG CGG TTC TGG AGC AG-3'
zwf-reverse (SEQ ID NO:11):
5'-CTA AAT TAT GGC CTG CGC CAG-3'
encoding a polypeptide having the activity of glucose-6-phosphate dehydrogenase, wherein said polypeptide contains the N-terminal amino acid sequence (SEQ ID NO:9) Ser Thr Asn Thr Thr Pro Ser Ser Trp Thr Asn Pro Leu Arg Asp.

The invention also provides L-lysine and L-threonine producing recombinant coryneform bacteria wherein said bacteria is transformed by the gene according to claim 1 encoding a polypeptide having the activity of glucose 6-phosphate dehydrogenase and containing the N-terminal amino acid sequence
(SEQ ID NO:9) Ser Thr Asn Thr Thr Pro Ser Ser Trp Thr Asn Pro Leu Arg Asp.

Said gene is preferably amplified by increasing the number of copies of said gene or said gene is linked to a promoter.

Another part of the invention is a process for the preparation of L-threonine and L-lysine by fermentation of coryneform bacteria, which comprises,
carrying out the following steps:
a) fermentation of said L-amino acid-producing bacteria in which at least the zwf gene encoding glucose-6-phosphate dehydrogenase according to claim 1 is amplified,
b) concentration of said L-amino acid in the medium or in the cells of the bacteria and
c) isolation of said L-amino acid produced.

The strains employed preferably already produce L-lysine or L-threonine, before amplification of the zwf gene.

Preferred embodiments are to be found in the claims.

The term "amplification" in this connection describes the increase in the intracellular activity of one or more enzymes (proteins) in a microorganism which are coded by the corresponding DNA, for example by increasing the number of copies of the gene or genes, using a potent promoter or using a gene which codes for a corresponding enzyme (protein) having a high activity, and optionally combining these measures.

The microorganisms which the present invention provides can prepare L-lysine and L-threonine from glucose, sucrose, lactose, fructose, maltose, molasses, starch, cellulose or from glycerol and ethanol. They are representatives of coryneform bacteria, in particular of the genus Corynebacterium. Of the genus Corynebacterium, there may be mentioned in particular the species Corynebacterium glutamicum, which is known among specialists for its ability to produce said L-amino acids.

Suitable strains of the genus Corynebacterium, in particular of the species Corynebacterium glutamicum, are, for example, the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869
Brevibacterium divaricatum ATCC14020
and L-amino acid-producing mutants prepared therefrom,
such as, for example, the L-threonine-producing strains
Corynebacterium glutamicum ATCC21649
Brevibacterium flavum BB69
Brevibacterium flavum DSM5399
Brevibacterium lactofermentum FERM-BP 269
Brevibacterium lactofermentum TBB-10 .

The description provides also, for example, the L-isoleucine-producing strains
Corynebacterium glutamicum ATCC 14309
Corynebacterium glutamicum ATCC 14310
Corynebacterium glutamicum ATCC 14311
Corynebacterium glutamicum ATCC 15168
Corynebacterium ammoniagenes ATCC 6871
and such as, for example, the L-tryptophan-producing strains
Corynebacterium glutamicum ATCC21850 and
Corynebacterium glutamicum KY9218(pKW9901)
and such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum ATCC13032
Corynebacterium glutamicum DM58-1
Corynebacterium glutamicum DSM12866.

It has been found that coryneform bacteria produce L-lysine and L-threonine, in an improved manner after over-expression of the zwf gene which codes for the Zwf protein.

Alleles of the zwf gene which result from the degeneracy of the genetic code or due to sense mutations of neutral function can furthermore be used.

JP-A-09224661 discloses the nucleotide sequence of the glucose 6-phosphate dehydrogenase gene, called zwf, of Brevibacterium flavum MJ-223 (FERM BP-1497). JP-A-09224661 describes the N-terminal amino acid sequence of the Zwf polypeptide as Met Val Ile Phe Gly Val Thr Gly Asp Leu Ala Arg Lys Lys Leu.

However, it has not been possible to confirm this. Instead, the following N-terminal amino acid sequence has been found: Val Ser Thr Asn Thr Thr Pro Ser Ser Trp Thr Asn Pro Leu Arg Asp. The valyl radical in the N-position can be split off in the context of post-translational modification, and Ser Thr Asn Thr Thr Pro Ser Ser Trp Thr Asn Pro Leu Arg Asp is then obtained as the N-terminal amino acid sequence.

To achieve an amplification (e. g. over-expression), the number of copies of the corresponding genes is increased, or the promoter and regulation region or the ribosome binding site upstream of the structural gene is mutated. Expression cassettes which are incorporated upstream of the structural gene act in the same way. By inducible promoters, it is additionally possible to increase the expression in the course of fermentative L-amino acid formation. The expression is likewise improved by measures to prolong the life of the m-RNA. Furthermore, the enzyme activity is also increased by preventing the degradation of the enzyme protein. The genes or gene constructs are either present here in plasmids with a varying number of copies, or are integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification EPS 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15-24 (1993)), in Japanese Laid-Open Specification JP-A-10-Z29891, in Jensen and Hammer (Biotechnology and Bioengineering 58, 191-195 (1998)) and in known textbooks of genetics and molecular biology.

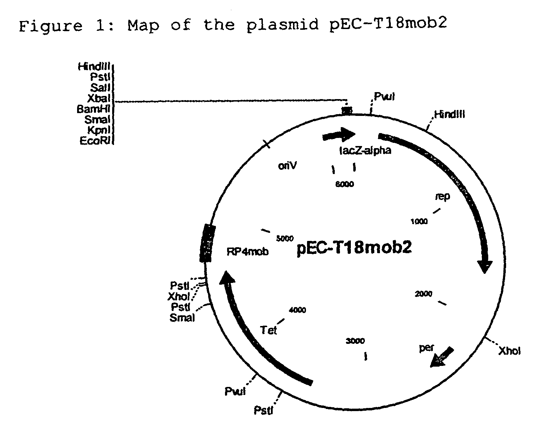

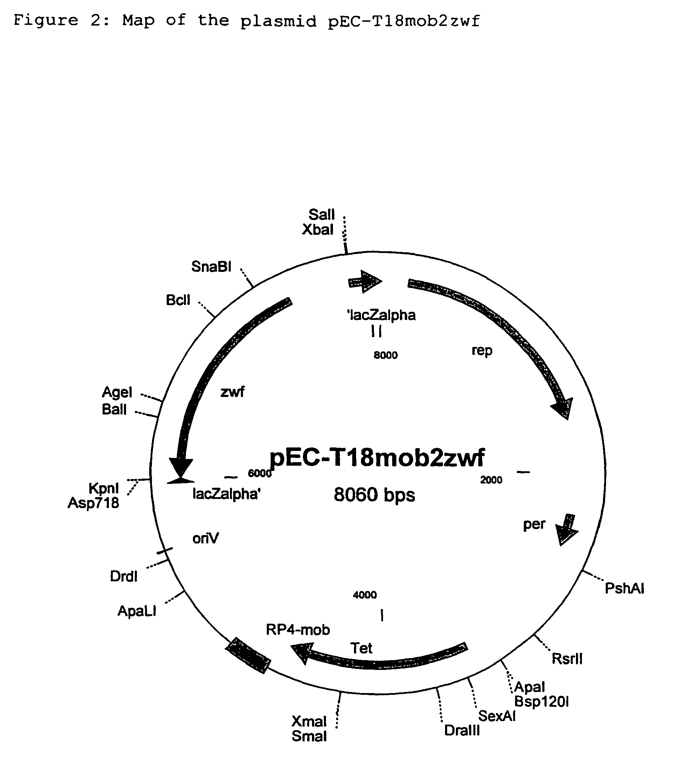

By way of example, the Zwf protein was over-expressed with the aid of a plasmid. The E. coli - C. glutamicum shuttle vector pEC-T18mob2 shown in Figure 1 was used for this. After incorporation of the zwf gene into the KpnI/SalI cleavage site of pEC-T18mob2, the plasmid pEC-T18mob2zwf shown in Figure 2 was formed.

Other plasmid vectors which are capable of replication in C. glutamicum, such as e.g. pEKEx1 (Eikmanns et al., Gene 102:93-98 (1991)) or pZ8-1 (EP-B- 0 375 889), can be used in the same way.

In addition, it may be advantageous for the production of L-lysine and L-threonine to amplify one or more enzymes of the particular biosynthesis pathway, of glycolysis, of anaplerosis, of the pentose phosphate pathway or of amino acid export, in addition to amplification of the zwf gene.

Thus, for example, in particular for the preparation of L-threonine, one or more genes chosen from the group consisting of:
- the hom gene which codes for homoserine dehydrogenase (Peoples et al., Molecular Microbiology 2, 63-72 (1988)) or the hom^{dr} allele which codes for a "feed back resistant" homoserine dehydrogenase (Archer et al., Gene 107, 53-59 (1991),
- the gap gene which codes for glyceraldehyde 3-phosphate dehydrogenase (Eikmanns et al., Journal of Bacteriology 174: 6076-6086 (1992)),
- the pyc gene which codes for pyruvate carboxylase (Peters-Wendisch et al., Microbiology 144: 915-927 (1998)),
- the mqo gene which codes for malate:quinone oxidoreductase (Molenaar et al., European Journal of Biochemistry 254, 395-403 (1998)),
- the tkt gene which codes for transketolase (accession number AB023377 of the European Molecular Biologies Laboratories databank (EMBL, Heidelberg, Germany)),
- the gnd gene which codes for 6-phosphogluconate dehydrogenase (JP-A-9-224662),
- the thrE gene which codes for threonine export (DE 199 41 478.5; DSM 12840),
- the zwa1 gene (DE 199 59 328.0; DSM 13115),
- the eno gene which codes for enolase (DE: 199 41 478.5)
can be amplified, in particular over-expressed, at the same time.

Thus, for example, in particular for the preparation of L-lysine, one or more genes chosen from the group consisting of
- the dapA gene which codes for dihydrodipicolinate synthase (EP-B 0 197 335),
- the lysC gene which codes for a feed back resistant aspartate kinase (Kalinowski et al. (1990), Molecular and General Genetics 224: 317-324),
- the gap gene which codes for glyceraldehyde 3-phosphate dehydrogenase (Eikmanns (1992), Journal of Bacteriology 174:6076-6086),
- the pyc gene which codes for pyruvate carboxylase (DE-A-198 31 609),
- the tkt gene which codes for transketolase (accession number AB023377 of the European Molecular Biologies Laboratories databank (EMBL, Heidelberg, Germany)), <<
- the gnd gene which codes for 6-phosphogluconate dehydrogenase (JP-A-9-224662),
- the lysE gene which codes for lysine export (DE-A-195 48 222),
- the zwa1 gene (DE 199 59 328.0; DSM 13115),
- the eno gene which codes for enolase (DE 199 47 791.4)
can be amplified, in particular over-expressed, at the same time.

It may furthermore be advantageous for the production of L-lysine and L-threonine at the same time to attenuate one of the genes chosen from the group consisting of
- the pck gene which codes for phosphoenol pyruvate carboxykinase (DE 199 50 409.1; DSM 13047),
- the pgi gene which codes for glucose 6-phosphate isomerase (EP 1087015 A, DSM 12969),
- the poxB gene which codes for pyruvate oxidase (DE 199 51 975.7; DSM 13114),
- the zwa2 gene (DE: 199 59 327.2; DSM 13113)
in addition to the amplification of the zwf gene.

In addition to over-expression of the Zwf protein, it may furthermore be advantageous for the production of said L-amino acids to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The microorganisms prepared according to the invention can be cultured continuously or discontinuously in the batch process (batch culture) or in the fed batch (feed process) or repeated fed batch process (repetitive feed process) for the purpose of L-lysine and L-threonine production. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular microorganisms in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as e. g. soya oil, sunflower oil, groundnut oil and coconut fat, fatty acids, such as e. g. palmitic acid, stearic acid and linoleic acid, alcohols, such as e. g. glycerol and ethanol, and organic acids, such as e. g. acetic acid, can be used as the source of carbon. These substance can be used individually or as a mixture. Organic nitrogen-containing compounds, such as peptones, yeast extract, meat extract, malt extract, corn steep liquor, soya bean flour and urea, or inorganic compounds, such as ammonium sulphate, ammonium chloride, ammonium phosphate, ammonium carbonate and ammonium nitrate, can be used as the source of nitrogen. The sources of nitrogen can be used individually or as a mixture. Potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of L-amino acid has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30, (1958), 1190), or it can take place by reversed phase HPLC as described by Lindroth et al. (Analytical Chemistry (1979) 51:. 1167-1174).

The following microorganism has been deposited at the Deutsche Sammlung für Mikroorganismen und Zellkulturen (DSMZ = German Collection of Microorganisms and Cell Cultures, Braunschweig, Germany) in accordance with the Budapest Treaty:
Escherichia coli K-12 DH5α/pEC-T18mob2 as DSM 13244

The following figures are attached:
- Figure 1: Map of the plasmid pEC-T18mob2
- Figure 2: Map of the plasmid pEC-T18mob2zwf
- Figure 3: Map of the plasmid pAMC1
- Figure 4: Map of the plasmid pMC1
- Figure 5: Map of the plasmid pCR2.1poxBint

The base pair numbers stated are approx. values obtained in the context of reproducibility.

### Re Figure 1 and 2:

The abbreviations used have the following meaning:

| | |
|---|---|
| Tet: | Resistance gene for tetracycline |
| oriV: | Plasmid-coded replication origin of E. coli |
| RP4mob: | mob region for mobilizing the plasmid |
| rep: | Plasmid-coded replication origin from C. glutamicum plasmid pGA1 |
| per: | Gene for controlling the number of copies from pGA1 |
| lacZ-alpha: | lacZα gene fragment (N-terminus) of the β-galactosidase gene |
| lacZalpha': | 5'-Terminus of the lacZα gene fragment |
| 'lacZalpha: | 3'-Terminus of the lacZα gene fragment |

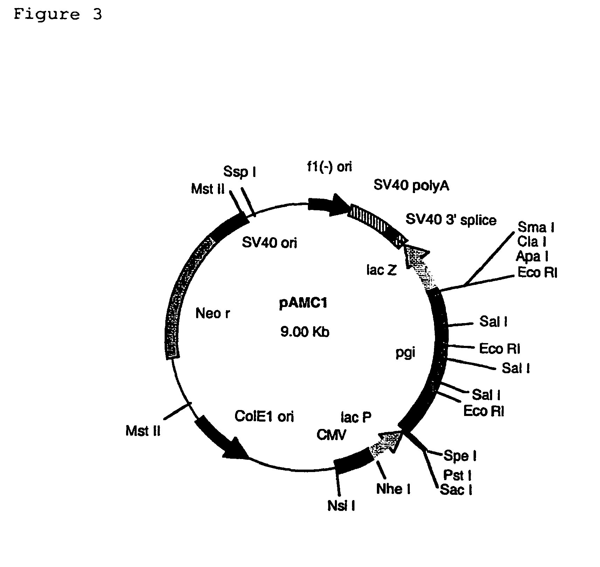

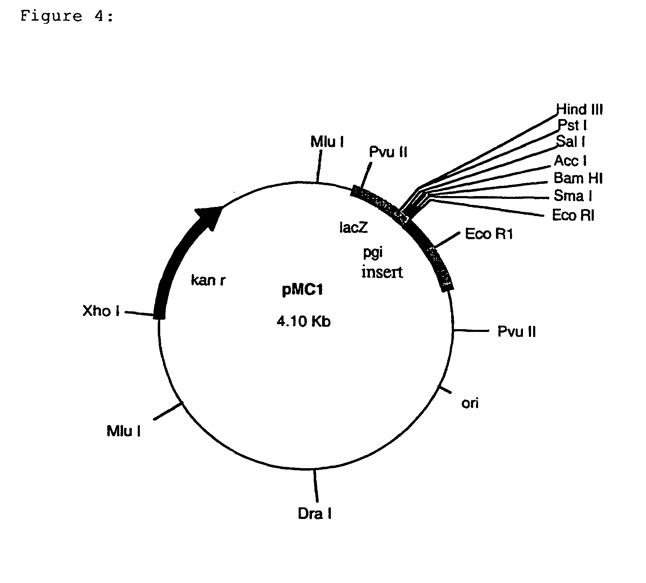

### Re Figure 3 and 4:

The abbreviations used have the following meaning:

| | |
|---|---|
| Neo r : | Neomycin/kanamycin resistance |
| ColE1 ori: | Replication origin of the plasmid ColE1 |
| CMV: | Cytomegalovirus promoter |
| lacP: | Lactose promoter |
| pgi: | Phosphoglucose isomerase gene |
| lacZ: | Part of the β-galactosidase gene |
| SV40 3' splice | 3' splice site of Simian virus 40 |
| SV40 polyA: | Polyadenylation site of Simian virus 40 |
| f1(-)ori: | Replication origin of the filamentous phage f1 |
| SV40 ori: | Replication origin of Simian virus 40 |
| kan r: | Kanamycin resistance |
| pgi insert: | Internal fragment of the pgi gene |
| ori: | Replication origin of the plasmid pBGS8 |

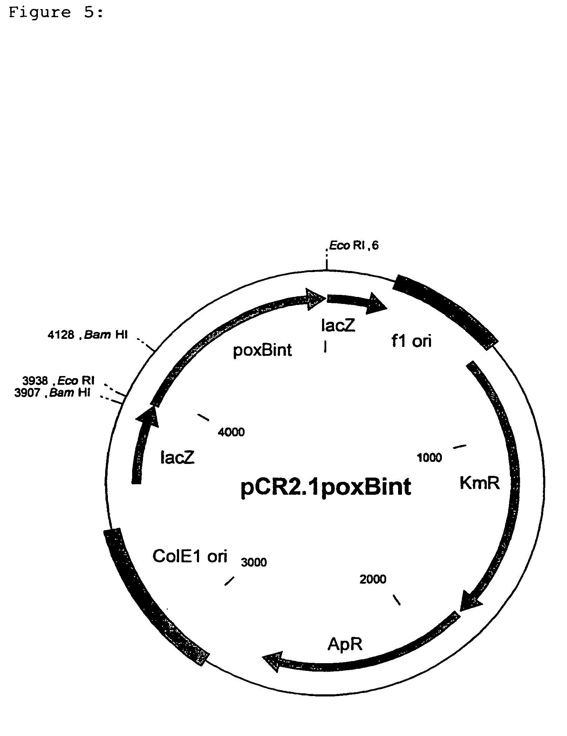

### Re Figure 5:

The abbreviations used have the following meaning:

| | |
|---|---|
| ColE1 ori: | Replication origin of the plasmid ColE1 |
| lacZ: | Cloning relict of the lacZα gene fragment |
| fl ori: | Replication origin of phage f1 |
| KmR: | Kanamycin resistance |
| ApR: | Ampicillin resistance |
| poxBint: | Internal fragment of the poxB gene |

The meaning of the abbreviations for the various restriction enzymes (e. g. BamHI, EcoRI etc.)are known from the prior art and are summarized, for example, by Kessler and Höltke (Gene 47, 1-153 (1986)) or Roberts et al. (Nucleic Acids Research 27, 312-313 (1999)).

### Examples

The following examples will further illustrate this invention. The molecular biology techniques, e.g. plasmid DNA isolation, restriction enzyme treatment, ligations, standard transformations of Escherichia coli etc. used are, (unless stated otherwise), described by Sambrook et al., (Molecular Cloning. A Laboratory Manual (1989) Cold Spring Harbour Laboratories, USA).

### Example 1

### Expression of the Zwf protein

### 1.1 Preparation of the plasmid pEC-T18mob2

The E. coli - C. glutamicum shuttle vector pEC-T18mob2 was constructed according to the prior art. The vector contains the replication region rep of the plasmid pGA1 including the replication effector per (US-A- 5,175,108; Nesvera et al., Journal of Bacteriology 179, 1525-1532 (1997)), the tetracycline resistance-imparting tetA(Z) gene of the plasmid pAG1 (US-A- 5,158,891; gene library entry at the National Center for Biotechnology Information (NCBI, Bethesda, MD, USA) with accession number AF121000), the replication region oriV of the plasmid pMB1 (Sutcliffe, Cold Spring Harbor Symposium on Quantitative Biology 43, 77-90 (1979)), the lacZα gene fragment including the lac promoter and a multiple cloning site (mcs) (Norrander et al. Gene 26, 101-106 (1983)) and the mob region of the plasmid RP4 (Simon et al.,(1983) Bio/Technology 1:784-791). The vector constructed was transformed in the E. coli strain DH5α (Brown (ed.) Molecular Biology Labfax, BIOS Scientific Publishers, Oxford, UK, 1991). Selection for plasmid-carrying cells was made by plating out the transformation batch on LB agar (Sambrook et al., Molecular cloning: a laboratory manual. 2^{nd} Ed. Cold Spring Harbor Laboratory Press, Cold Spring Harbor, N.Y.), which had been supplemented with 5 mg/l tetracycline. Plasmid DNA was isolated from a transformant with the aid of the QIAprep Spin Miniprep Kit from Qiagen and checked by restriction with the restriction enzyme EcoRI and HindIII and subsequent agarose gel electrophoresis (0.8%).

The plasmid was called pEC-T18mob2 and is shown in Figure 1. It is deposited in the form of the strain Escherichia coli K-12 strain DH5αpEC-T18mob2 at the Deutsche Sammlung für Mikroorganismen und Zellkulturen (DSMZ = German Collection of Microorganisms and Cell Cultures, Braunschweig, Germany) as DSM 13244.

### 1.2 Preparation of the plasmid pEC-T18mob2zwf

The gene from Corynebacterium glutamicum ATCC13032 was first amplified by a polymerase chain reaction (PCR) by means of the following oligonucleotide primer:
SEQ ID NO:10
zwf-forward:
5'-TCG ACG CGG TTC TGG AGC AG-3'
SEQ ID NO:11
zwf-reverse:
5'-CTA AAT TAT GGC CTG CGC CAG-3'

The PCR reaction was carried out in 30 cycles in the presence of 200 µM deoxynucleotide triphosphates (dATP, dCTP, dGTP, dTTP), in each case 1 µM of the corresponding oligonucleotide, 100 ng chromosomal DNA from Corynebacterium glutamicum ATCC13032, 1/10 volume 10-fold reaction buffer and 2.6 units of a heat-stable Taq-/Pwo-DNA polymerase mixture (Expand High Fidelity PCR System from Roche Diagnostics, Mannheim, Germany) in a Thermocycler (PTC-100, MJ Research, Inc., Watertown, USA) under the following conditions: 94°C for 30 seconds, 64°C for 1 minute and 68°C for 3 minutes.

The amplified fragment about 1.8 kb in size was subsequently ligated with the aid of the SureClone Ligation Kit (Amersham Pharmacia Biotech, Uppsala, Sweden) into the SmaI cleavage site of the vector pUC18 in accordance with the manufacturer's instructions. The E. coli strain DH5α mcr (Grant et al., Proceedings of the National Academy of Sciences of the United States of America USA (1990) 87: 4645-4649) was transformed with the entire ligation batch. Transformants were identified with the aid of their carbenicillin resistance on LB-agar plates containing 50 µg/mL carbenicillin. The plasmids were prepared from 7 of the transformants and checked for the presence of the 1.8 kb PCR fragment as an insert by restriction analysis. The recombinant plasmid formed in this way is called pUC18zwf in the following.

For construction of pEC-T18mob2zwf, pUC18zwf was digested with KpnI and SalI, and the product was isolated with the aid of the NucleoSpin Extraction Kit from Macherey-Nagel (Düren, Germany) in accordance with the manufacturer's instructions and then ligated with the vector pEC-T18mob2, which had also been cleaved with KpnI and SalI and dephosphorylated. The E. coli strain DH5αmcr (Grant et al., Proceedings of the National Academy of Sciences of the United States of America USA (1990) 87: 4645-4649) was transformed with the entire ligation batch. Transformants were identified with the aid of their tetracycline resistance on LB-agar plates containing 5 µg/mL tetracycline. The plasmids were prepared from 12 of the transformants and checked for the presence of the 1.8 kb PCR fragment as an insert by restriction analysis. One of the recombinant plasmids isolated in this manner was called pEC-T18mob2zwf (Figure 2).

### Example 2

### Preparation of amino acid producers with an amplified zwf gene

The L-lysine-producing strain Corynebacterium glutamicum DSM5715 is described in EP-B-0435132 and the L-threonine-producing strain Brevibacterium flavum DSM5399 is described in EP-B-0385940. Both strains are deposited at the Deutsche Sammlung für Mikroorganismen und Zellkulturen [German Collection of Microorganisms and Cell Cultures] in Braunschweig (Germany) in accordance with the Budapest Treaty.

### 2.1 Preparation of the strains DSM5715/pEC-T18mob2zwf and DSM5399/pEC-T18mob2zwf

The strains DSM5715 and DSM5399 were transformed with the plasmid pEC-T18mob2zwf using the electroporation method described by Liebl et al., (FEMS Microbiology Letters, 53:299-303 (1989)) Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bactoagar, which had been supplemented with 5 mg/l tetracycline. Incubation was carried out for 2 days at 33°C.

Plasmid DNA was isolated in each case from a transformant by conventional methods (Peters-Wendisch et al., 1998, Microbiology 144, 915 -927), cleaved with the restriction endonucleases XbaI and KpnI, and the plasmid was checked by subsequent agarose gel electrophoresis. The strains obtained in this way were called DSM5715/pEC-T18mob2zwf and DSM5399/pEC-T18mob2zwf.

### 2.2 Preparation of L-threonine

The C. glutamicum strain DSM5399/pEC-T18mob2zwf obtained in Example 2.1 was cultured in a nutrient medium suitable for the production of threonine and the threonine content in the culture supernatant was determined.

For this, the strain was first incubated on an agar plate with the corresponding antibiotic (brain-heart agar with tetracycline (5 mg/l)) for 24 hours at 33°C. Starting from this agar plate culture, a preculture was seeded (10 ml medium in a 100 ml conical flask). The complete medium CgIII was used as the medium for the preculture.

### Medium Cg III

| | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) was added to this. The preculture was incubated for 16 hours at 33°C at 240 rpm on a shaking machine. A main culture was seeded from this preculture such that the initial OD (660nm) of the main culture was 0.1. Medium MM was used for the main culture.

### Medium MM

| | |
|---|---|
| CSL (corn steep liquor) | 5 g/l |
| MOPS (morpholinopropanesulfonic acid) | 20 g/l |
| Glucose (autoclaved separately) | 50 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0 mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) was added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of threonine formed was determined with an amino acid analyzer from Eppendorf-BioTronik (Hamburg, Germany) by ion exchange chromatography and post-column derivatization with ninhydrin detection.

The result of the experiment is shown in Table 1.

**Table 1**

| Strain | OD | L-Threonin g/l |
|---|---|---|
| DSM5399 | 12.3 | 0.74 |
| DSM5399/pEC-T18mob2zwf | 10.2 | 1.0 |

### 2.3 Preparation of L-lysine

The C. glutamicum strain DSM5715/pEC-T18mob2zwf obtained in Example 2.1 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l)) for 24 hours at 33°C. Starting from this agar plate culture, a preculture was seeded (10 ml medium in a 100 ml conical flask). The complete medium CgIII was used as the medium for the preculture.

### Medium Cg III

| | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) was added to this. The preculture was incubated for 16 hours at 33°C at 240 rpm on a shaking machine. A main culture was seeded from this preculture such that the initial OD (660nm) of the main culture was 0.1. Medium MM was used for the main culture.

### Medium MM

| | |
|---|---|
| CSL (corn steep liquor) | 5 g/l |
| MOPS (morpholinopropanesulfonic acid) | 20 g/l |
| Glucose (autoclaved separately) | 58 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) was added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 2.

**Table 2**

| Strain | OD | L-Lysine HCl g/l |
|---|---|---|
| DSM5715 | 10.8 | 16.0 |
| DSM5715/pEC-T18mob2zwf | 7.2 | 17.1 |

### Example 3

### Construction of a gene library of Corynebacterium glutamicum strain AS019

A DNA library of Corynebacterium glutamicum strain ASO19 (Yoshihama et al., Journal of Bacteriology 162, 591-597 (1985)) was constructed using λ Zap Express^{™} system, (Short et al., (1988) Nucleic Acids Research, 16: 7583-7600), as described by O'Donohue (O'Donohue, M. (1997). The Cloning and Molecular Analysis of Four Common Aromatic Amino Acid Biosynthetic Genes from Corynebacterium glutamicum. Ph.D. Thesis, National University of Ireland, Galway). λ Zap Express^{™} kit was purchased from Stratagene (Stratagene, 11011 North Torrey Pines Rd., La Jolla, California 92037) and used according to the manufacturers instructions. AS019-DNA was digested with restriction enzyme Sau3A and ligated to BamHI treated and dephosphorylated λ Zap Express^{™} arms.

### Example 4

### Cloning and sequencing of the pgi gene

### 1. Cloning

Escherichia coli strain DF1311, carrying mutations in the pgi and pgl genes as described by Kupor and Fraenkel, (Journal of Bacteriology 100: 1296-1301 (1969)), was transformed with approx. 500 ng of the AS019 λ Zap Express^{™} plasmid library described in Example 3. Selection for transformants was made on M9 minimal media, (Sambrook et al., (1989). Molecular Cloning. A Laboratory Manual Cold Spring Harbour Laboratories, USA), containing kanamycin at a concentration of 50 mg/l and incubation at 37°C for 48 hours. Plasmid DNA was isolated from one transformant according to Birnboim and Doly (Nucleic Acids Research 7: 1513-1523 (1979)) and designated pAMC1 (Figure 3).

### 2. Sequencing

For sequence analysis of the cloned insert of pAMC1 the method of Sanger et al. (Proceedings of the National Academy of Sciences USA 74,5463-5467 (1977)) was applied using primers differentially labelled with a coloured fluorescent tag. It was carried out using the ABI prism 310 genetic analyzer from Perkin Elmer Applied Biosystems, (Perkin Elmer Corporation, Norwalk, Connecticut, U.S.A), and the ABI prism Big Dye^{™} Terminator Cycle Sequencing Ready Reaction kit also from Perkin Elmer.

Initial sequence analysis was carried out using the universal forward and M13 reverse primers obtained from Pharmacia Biotech (St. Albans, Herts, AL1 3AW, UK):
Universal forward primer: GTA ATA CGA CTC ACT ATA GGG C
M13 reverse primer: GGA AAC AGC TAT GAC CAT G
Internal primers were subsequently designed from the sequence obtained which allowed the entire pgi gene to be deduced. The sequence of the internal primers is as follows:
Internal primer 1: GGA AAC AGG GGA GCC GTC
Internal primer 2: TGC TGA GAT ACC AGC GGT

Sequence obtained was then analyzed using the DNA Strider programme, (Marck, (1988). Nucleic Acids Research 16: 1829-1836), version 1.0 on an Apple Macintosh computer. This program allowed for analyses such as restriction site usage, open reading frame analysis and codon usage determination. Searches between DNA sequence obtained and those in EMBL and Genbank databases were achieved using the BLAST programme, (Altschul et al., (1997). Nucleic Acids Research, 25: 3389-3402). DNA and protein sequences were aligned using the Clustal V and Clustal W programs (Higgins and Sharp, 1988 Gene 73: 237-244).

The sequence thus obtained is shown in SEQ ID NO 1. The analysis of the nucleotide sequence obtained revealed an open reading frame of 1650 base pairs which was designated as pgi gene. It codes for a protein of 550 amino acids shown in SEQ ID NO 2.

### Example 5

### Preparation of an integration vector for integration mutagenesis of the pgi gene

An internal segment of the pgi gene was amplified by polymerase chain reaction (PCR) using genomic DNA isolated from Corynebacterium glutamicum AS019, (Heery and Dunican, (1993) Applied and Environmental Microbiology 59: 791-799), as template. The pgi primers used were:
fwd. Primer: ATG GAR WCC AAY GGH AA
rev. Primer: YTC CAC GCC CCA YTG RTC
with R=A+G; Y=C+T; W=A+T; H=A+T+C.

| | |
|---|---|
| PCR Parameters were as follows: | 35 cycles |
| | 94°C for 1 min. |
| | 47°C for 1 min. |
| | 72°C for 30 sec. |
| | 1.5 mM MgCl₂ |
| | approx. 150-200 ng DNA template. |

The PCR product obtained was cloned into the commercially available pGEM-T vector received from Promega Corp., (Promega UK, Southampton.) using strain E. coli JM109, (Yanisch-Perron et al., 1985. Gene, 33: 103-119), as a host. The sequence of the PCR product is shown as SEQ ID NO 3. The cloned insert was then excised as an EcoRI fragment and ligated to plasmid pBGS8 (Spratt et al., Gene 41: 337-342 (1986)) pretreated with EcoRI. The restriction enzymes used were obtained from Boehringer Mannheim UK Ltd., (Bell Lane, Lewes East Sussex BN7 1LG, UK.) and used according to manufacturers instructions. E. coli JM109 was then transformed with this ligation mixture and electrotransformants were selected on Luria agar supplemented with IPTG (isopropyl-β-D-thiogalactopyranoside), XGAL (5-bromo-4-chloro-3-indolyl-D-galactopyranoside) and kanamycin at a concentration of 1 mM, 0.02% and 50 mg/l respectively. Agar plates were incubated for twelve hours at 37°C. Plasmid DNA was isolated from one transformant, characterized by restriction enzyme analysis using EcoRI, BamHI and SalI designated pMC1 (Figure 4).

Plasmid pMC1 was deposited in the form of Escherichia coli strain DH5a/pMC1 at the Deutsche Sammlung für Mikroorganismen und Zellkulturen (DSMZ, Braunschweig, Germany) as DSM 12969 according to the Budapest treaty.

### Example 6

### Integration mutagenesis of the pgi gene in the lysine producer DSM 5715

The vector pMC1 mentioned in Example 5 was electroporated by the electroporation method of Tauch et al. (FEMS Microbiological Letters, 123:343-347 (1994)) in Corynebacterium glutamicum DSM 5715. Strain DSM 5715 is an AEC-resistant lysine producer. The vector pMC1 cannot replicate independently in DSM5715 and is retained in the cell only if it has integrated into the chromosome of DSM 5715. Selection of clones with pMC1 integrated into the chromosome was carried out by plating out the electroporation batch on LB agar (Sambrook et al., Molecular cloning: a laboratory manual. 2^{nd} Ed. Cold Spring Harbor Laboratory Press, Cold Spring Harbor, N.Y.), which had been supplemented with 15 mg/l kanamycin. For detection of the integration, the internal pgi fragment (Example 5) was labelled with the Dig hybridization kit from Boehringer Mannheim by the method of "The DIG System Users Guide for Filter Hybridization" of Boehringer Mannheim GmbH (Mannheim, Germany, 1993). Chromosomal DNA of a transformant was isolated by the method of Eikmanns et al. (Microbiology 140: 1817 - 1828 (1994)) and in each case cleaved with the restriction enzymes SalI, SacI and HindIII. The fragments formed were separated by agarose gel electrophoresis and hybridized at 68°C with the Dig hybrization kit from Boehringer. It was found in this way that the plasmid pMC1 was inserted within the chromosomal pgi gene of strain DSM5715. The strain was called DSM5715::pMC1.

### Example 7

### Effect of over-expression of the zwf gene with simultaneous elimination of the pgi gene on the preparation of lysine

### 7.1 Preparation of the strain DSM5715::pMC1/pEC-T18mob2zwf

The vector pEC-T18mob2zwf mentioned in Example 1.2 was electroporated by the electroporation method of Tauch et al. (1994, FEMS Microbiological Letters, 123:343-347) in Corynebacterium glutamicum DSM 5715::pMC1. Selection for plasmid-carrying cells was made by plating out the electroporation batch on LB agar (Sambrook et al., Molecular cloning: a laboratory manual. 2^{nd} Ed. Cold Spring Harbor Laboratory Press, Cold Spring Harbor, N.Y., 1989), which had been supplemented with 15 mg/l kanamycin and with 5 mg/l tetracycline. Plasmid DNA was isolated from a transformant by conventional methods (Peters-Wendisch et al., 1998, Microbiology 144, 915-927) and checked by treatment with the restriction enzymes KpnI and SalI with subsequent agarose gel electrophoresis. The strain was called DSM5715::pMC1/pEC-T18mob2zwf.

### 7.2 Preparation of lysine

The C. glutamicum strain DSM5715::pMC1/pEC-T18mob2zwf obtained in Example 7.1 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l) and kanamycin (25 mg/l)) for 24 hours at 33°C. The cultures of the comparison strains were supplemented according to their resistance to antibiotics. Starting from this agar plate culture, a preculture was seeded (10 ml medium in a 100 ml conical flask). The complete medium CgIII was used as the medium for the preculture.

### Medium Cg III

| | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) and kanamycin (5 mg/l) was added to this. The preculture was incubated for 16 hours at 33°C at 240 rpm on a shaking machine. A main culture was seeded from this preculture such that the initial OD (660 nm) of the main culture was 0.1. Medium MM was used for the main culture.

### Medium MM

| | |
|---|---|
| CSL (corn steep liquor) | 5 g/l |
| MOPS (morpholinopropanesulfonic acid) | 20 g/l |
| Glucose (autoclaved separately) | 50 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) and kanamycin (25 mg/l) were added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 3.

**Table 3**

| Strain | OD | L-Lysine HCl g/l |
|---|---|---|
| DSM5715 | 7.3 | 14.3 |
| DSM5715/pEC-T18mob2zwf | 7.1 | 14.6 |
| DSM5715::pMC1/ pECTmob2zwf | 10.4 | 15.2 |

### Example 8

### Preparation of a genomic cosmid gene library from Corynebacterium glutamicum ATCC 13032

Chromosomal DNA from Corynebacterium glutamicum ATCC 13032 was isolated as described by Tauch et al., (1995, Plasmid 33:168-179), and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vektor Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase. The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extracts (Stratagene, La Jolla, USA, Product Description Gigapack II XL Packing Extract, Code no. 200217). For infection of the E. coli strain NM554 (Raleigh et al. 1988, Nucleic Acid Res. 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) + 100 µg/ml ampicillin. After incubation overnight at 37°C, recombinant individual clones were selected.

### Example 9

### Isolation and sequencing of the poxB gene

The cosmid DNA of an individual colony (Example 7) was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany). The DNA of the sequencing vector pZero-1, obtained from Invitrogen (Groningen, Holland, Product Description Zero Background Cloning Kit, Product No. K2500-01), was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology, 1:190) with 50 µg/ml zeocin. The plasmid preparation of the recombinant clones was carried out with Biorobot 9600 (Product No. 900200, Qiagen, Hilden, Germany). The sequencing was carried out by the dideoxy chain-stopping method of Sanger et al. (1977, Proceedings of the National Academies of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems(Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analysis were prepared with the XNIP program (Staden, 1986, Nucleic Acids Research, 14:217-231). Further analyses were carried out with the "BLAST search program" (Altschul et al., 1997, Nucleic Acids Research, 25:3389-3402), against the non-redundant databank of the "National Center for Biotechnology Information" (NCBI, Bethesda, MD, USA).

The resulting nucleotide sequence is shown in SEQ ID No. 4. Analysis of the nucleotide sequence showed an open reading frame of 1737 base pairs, which was called the poxB gene. The poxB gene codes for a polypeptide of 579 amino acids (SEQ ID NO. 5).

### Example 10

### Preparation of an integration vector for integration mutagenesis of the poxB gene

From the strain ATCC 13032, chromosomal DNA was isolated by the method of Eikmanns et al. (Microbiology 140: 1817 - 1828 (1994)). On the basis of the sequence of the poxB gene known for C. glutamicum from Example 8, the following oligonucleotides were chosen for the polymerase chain reaction:
poxBint1:
5' TGC GAG ATG GTG AAT GGT GG 3'
poxBint2:
5' GCA TGA GGC AAC GCA TTA GC 3'

The primers shown were synthesized by MWG Biotech (Ebersberg, Germany) and the PCR reaction was carried out by the standard PCR method of Innis et al. (PCR protocols. A guide to methods and applications, 1990, Academic Press) with Pwo-Polymerase from Boehringer. With the aid of the polymerase chain reaction, a DNA fragment approx. 0.9 kb in size was isolated, this carrying an internal fragment of the poxB gene and being shown in SEQ ID No. 6.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991) Bio/Technology 9:657-663). The E. coli strain DH5α was then electroporated with the ligation batch (Hanahan, In: DNA cloning. A practical approach. Vol.I. IRL-Press, Oxford, Washington DC, USA, 1985). Selection for plasmid-carrying cells was made by plating out the transformation batch on LB agar (Sambrook et al., Molecular cloning: a laboratory manual. 2^{nd} Ed. Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.1poxBint (Figure 5).

Plasmid pCR2.1poxBint has been deposited in the form of the strain Escherichia coli DH5α/pCR2.1poxBint as DSM 13114 at the Deutsche Sammlung für Mikroorganismen und Zellkulturen (DSMZ = German Collection of Microorganisms and Cell Cultures, Braunschweig, Germany) in accordance with the Budapest Treaty.

### Example 11

### Integration mutagenesis of the poxB gene in the lysine producer DSM 5715

The vector pCR2.1poxBint mentioned in Example 10 was electroporated by the electroporation method of Tauch et al.(FEMS Microbiological Letters, 123:343-347 (1994)) in Corynebacterium glutamicum DSM 5715. Strain DSM 5715 is an AEC-resistant lysine producer. The vector pCR2.1poxBint cannot replicate independently in DSM5715 and is retained in the cell only if it has integrated into the chromosome of DSM 5715. Selection of clones with pCR2.1poxBint integrated into the chromosome was carried out by plating out the electroporation batch on LB agar (Sambrook et al., Molecular cloning: a laboratory manual. 2^{nd} Ed. Cold Spring Harbor Laboratory Press, Cold Spring Harbor, N.Y.), which had been supplemented with 15 mg/l kanamycin. For detection of the integration, the poxBint fragment was labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes SalI, SacI and HindIII. The fragments formed were separated by agarose gel electrophoresis and hybridized at 68°C with the Dig hybrization kit from Boehringer. The plasmid pCR2.1poxBint mentioned in Example 9 had been inserted into the chromosome of DSM5715 within the chromosomal poxB gene. The strain was called DSM5715::pCR2.1poxBint.

### Example 12

### Effect of over-expression of the zwf gene with simultaneous elimination of the poxB gene on the preparation of lysine

### 12.1 Preparation of the strain DSM5715::pCR2.1poxBint/pEC-T18mob2zwf

The strain DSM5715::pCR2.1poxBint was transformed with the plasmid pEC-T18mob2zwf using the electroporation method described by Liebl et al., (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bactoagar, which had been supplemented with 5 mg/l tetracycline and 25 mg/l kanamycin. Incubation was carried out for 2 days at 33°C.

Plasmid DNA was isolated in each case from a transformant by conventional methods (Peters-Wendisch et al., 1998, Microbiology 144, 915-927), cleaved with the restriction endonucleases XbaI and KpnI, and the plasmid was checked by subsequent agarose gel electrophoresis. The strain obtained in this way was called DSM5715:pCR2.1poxBint/pEC-T18mob2zwf.

### 12.2 Preparation of L-lysine

The C. glutamicum strain DSM5715::pCR2.1poxBint/pEC-T18mob2zwf obtained in Example 12.1 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l) and kanamycin (25 mg/l)) for 24 hours at 33°C.The comparison strains were supplemented according to their resistance to antibiotics. Starting from this agar plate culture, a preculture was seeded (10 ml medium in a 100 ml conical flask). The complete medium CgIII was used as the medium for the preculture.

### Medium Cg III

| | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) and kanamycin (25 mg/l) were added to this. The preculture was incubated for 16 hours at 33°C at 240 rpm on a shaking machine. A main culture was seeded from this preculture such that the initial OD (660nm) of the main culture was 0.1. Medium MM was used for the main culture.

### Medium MM

| | |
|---|---|
| CSL (corn steep liquor) | 5 g/l |
| MOPS (morpholinopropanesulfonic acid) | 20 g/l |
| Glucose (autoclaved separately) | 58 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) and kanamycin (25 mg/l) were added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 4.

**Table 4**

| Strain | OD | L-Lysine HCl g/l |
|---|---|---|
| DSM5715 | 10.8 | 16.0 |
| DSM5715/pEC-T18mob2zwf | 8.3 | 17.1 |
| DSM5715::pCR2.1poxBint | 7.1 | 16.7 |
| DSM5715::pCR2.1poxBint/ pEC-Tmob2zwf | 7.8 | 17.7 |

## Claims

1. An isolated gene of Corynebacterium glutamicum (zwf gene) obtainable by PCR from C. glutamicum ATCC13032 by means of the oligonucleotide primer
zwf-forward (SEQ ID NO:10):
5'-TCG ACG CGG TTC TGG AGC AG-3'
zwf-reverse (SEQ ID NO:11) :
5'-CTA AAT TAT GGC CTG CGC CAG-3'
encoding a polypeptide having the activity of glucose-6-phosphate dehydrogenase, wherein said polypeptide contains the N-terminal amino acid sequence (SEQ ID NO:9) Ser Thr Asn Thr Thr Pro Ser Ser Trp Thr Asn Pro Leu Arg Asp.

2. L-lysine or L-threonine producing recombinant coryneform bacteria wherein said bacteria is transformed by the gene according to claim 1 encoding a polypeptide having the activity of glucose 6-phosphate dehydrogenase and containing the N-terminal amino acid sequence (SEQ ID NO:9) Ser Thr Asn Thr Thr Pro Ser Ser Trp Thr Asn Pro Leu Arg Asp.

3. Coryneform bacteria according to claim 2 wherein said gene is amplified by increasing the number of copies of said gene.

4. Coryneform bacteria according to claim 2, wherein said gene is linked to a promotor.

5. A process for the preparation of L-lysine or L-threonine by fermentation of coryneform bacteria, which comprises, carrying out the following steps:
a) fermentation of said L-amino acid-producing bacteria in which at least the zwf gene encoding glucose-6-phosphate dehydrogenase according to claim 1 is amplified,
b) concentration of said L-amino acid in the medium or in the cells of the bacteria and
c) isolation of said L-amino acid produced.

6. The process according to claim 5, wherein coryneform bacteria which prepare L-threonine or L-lysine are used.

7. The process according to claim 6, wherein bacteria of the genus Corynebacterium which prepare L-lysine are used.

8. A process for the fermentative preparation of L-lysine according to claim 5, wherein in the coryneform bacteria one or more genes chosen from the group consisting of
a) the dapA gene which codes for dihydrodipicolinate synthase,
b) the lysC gene which codes for a feed back resistant aspartate kinase,
c) the gap gene which codes for glycerolaldehyde 3-phosphate dehydrogenase,
d) the pyc gene which codes for pyruvate carboxylase,
e) the tkt gene which codes for transketolase,
f) the gnd gene which codes for glucose 6-phosphate dehydrogenase,
g) the lysE gene which codes for lysine export,
h) the gene, called zwa1 and deposited under DSM 13115,
i) the eno gene which codes for enolase
is or are amplified at the same time.

9. A process for the fermentative preparation of L-lysine according to claim 5, wherein in the coryneform bacteria one or more genes chosen from the group consisting of
a) the dapA gene which codes for dihydrodipicolinate synthase,
b) the lysC gene which codes for a feed back resistant aspartate kinase,
c) the gap gene which codes for glycerolaldehyde 3-phosphate dehydrogenase,
d) the pyc gene which codes for pyruvate carboxylase,
e) the tkt gene which codes for transketolase,
f) the gnd gene which codes for glucose 6-phosphate dehydrogenase,
g) the lysE gene which codes for lysine export,
h) the gene, called zwa1 and deposited under DSM 13115,
i) the eno gene which codes for enolase
is or are over-expressed at the same time.

10. A process for the fermentative preparation of L-threonine according to claim 5, wherein in the coryneform bacteria one or more genes chosen from the group consisting of
a) the hom gene which codes for homoserine dehydrogenase or the hom^{dr} allele which codes for a "feed back resistant" homoserine dehydrogenase,
b) the gap gene which codes for glycerolaldehyde 3-phosphate dehydrogenase,
c) the pyc gene which codes for pyruvate carboxylase,
d) the mqo gene which codes for malate:quinone oxidoreductase,
e) the tkt gene which codes for transketolase,
f) the gnd gene which codes for 6-phosphogluconate dehydrogenase,
g) the thrE gene which codes for threonine export,
h) the gene, called zwa1 and deposited under DSM 13115,
i) the eno gene which codes for enolase
is or are amplified at the same time.

11. A process for the fermentative preparation of L-threonine according to claim 5, wherein in the coryneform bacteria one or more genes chosen from the group consisting of
a) the hom gene which codes for homoserine dehydrogenase or the hom^{dr} allele which codes for a "feed back resistant" homoserine dehydrogenase,
b) the gap gene which codes for glycerolaldehyde 3-phosphate dehydrogenase,
c) the pyc gene which codes for pyruvate carboxylase,
d) the mqo gene which codes for malate:quinone oxidoreductase,
e) the tkt gene which codes for transketolase,
f) the gnd gene which codes for 6-phosphogluconate dehydrogenase,
g) the thrE gene which codes for threonine export,the gene called zwa1 and deposited under DSM 13115
h) the eno gene which codes for enolase
is or are over-expressed, at the same time.

12. The process according to claim 5, wherein for the preparation of L-lysine or L-threonine, bacteria in which one or more genes chosen from the group consisting of,
a) the pck gene which codes for phosphoenol pyruvate carboxykinase,
b) the pgi gene which codes for glucose 6-phosphate isomerase
c) the poxB gene which codes for pyruvate oxidase or
d) the gene, called zwa2 and deposited under DSM 13113
is or are attenuated at the same time, are fermented.

13. The process according to claim 5, wherein to achieve the amplification, the number of copies of the zwf gene according to claim 1 is increased by transformation of the bacteria with plasmid vectors which carry this gene.

14. The process according to claim 5 to 11 wherein to achieve the amplification of additional genes or nucleotide sequences, the number of copies of the said genes or nucleotide sequences is increased by transformation of the bacteria with plasmid vectors which carry said genes or nucleotide sequences.

15. The process according to claim 5, wherein alleles of the zwf gene according to claim 1, which result from the degeneracy of the genetic code are amplified.

16. The process according to claim 5, wherein alleles of the zwf gene according to claim 1 which are due to sense mutations of neutral sense are amplified.

## Patentansprüche

1. Aus Corynebacterium glutamicum isoliertes Gen (zwf-Gen), welches durch Anwendung von PCR auf C. glutamicum ATCC13032 mittels des folgenden Oligonucleotidprimers erhalten werden kann:
zwf-vorwärts (SEQ ID Nr.: 10):
5'-TCG ACG CGG TTC TGG AGC AG-3'
zwf-rückwärts (SEQ ID Nr.: 11):
5'-CTA AAT TAT GGC CTG CGC CAG-3'
und welches für ein Polypeptid codiert, das eine Glucose-6-phosphat-Dehydrogenase-Aktivität aufweist, wobei das Polypeptid die N-terminale Aminosäuresequenz (SEQ ID Nr.: 9) Ser Thr Asn Thr Pro Ser Ser Trp Thr Asn Pro Arg Asp enthält.

2. L-Lysin- oder L-Threonin produzierende rekombinante coryneforme Bakterien, wobei die Bakterien durch das Gen nach Anspruch 1, welches für ein Polypeptid codiert, das eine Glucose-6-phosphat-Dehydrogenase-Aktivität aufweist und die N-terminale Aminosäuresequenz (SEQ ID Nr.: 9) Ser Thr Asn Thr Pro Ser Ser Trp Thr Asn Pro Arg Asp enthält, verändert sind.

3. Coryneforme Bakterien nach Anspruch 2, wobei das Gen durch Erhöhen der Anzahl der Kopien dieses Gens verstärkt ist.

4. Coryneforme Bakterien nach Anspruch 2, wobei das Gen mit einem Promotor verbunden ist.

5. Verfahren zur Herstellung von L-Lysin oder L-Threonin durch Fermentieren von coryneformen Bakterien, welches das Ausführen folgender Schritte umfasst:
a) Fermentieren der L-Aminosäure produzierenden Bakterien, wobei mindestens das zwf-Gen gemäß Anspruch 1, welches für Glucose-6-phosphat-Dehydrogenase codiert, verstärkt ist.
b) Konzentrieren der L-Aminosäure im Medium oder in den Bakterienzellen sowie
c) Isolieren der produzierten L-Aminosäure.

6. Verfahren nach Anspruch 5, wobei coryneforme Bakterien, die L-Threonin oder L-Lysin herstellen, verwendet werden.

7. Verfahren nach Anspruch 6, wobei Bakterien der Gattung Corynebacterium, die L-Lysin herstellen, verwendet werden.

8. Verfahren zur fermentativen Herstellung von L-Lysin gemäß Anspruch 5, wobei in den coryneformen Bakterien eines oder mehrere Gene, die aus der Gruppe gewählt werden, die aus folgenden Genen besteht:
a) dapA-Gen, welches für Dihydrodipicolinat-Synthase codiert,
b) lysC-Gen, welches für eine rückkopplungsresistente Aspartat-Kinase codiert,
c) gap-Gen, welches für Glycerinaldehyd-3-phosphat-Dehydrogenase codiert,
d) pyc-Gen, welches für Pyruvat-Carboxylase codiert,
e) tkt-Gen, welches für Transketolase codiert,
f) gnd-Gen, welches für Glucose-6-phosphat-Dehydrogenase codiert,
g) lysE-Gen, welches für den Lysinexport codiert,
h) als zwa1 bezeichnetes Gen, welches unter der Nummer DSM 13115 hinterlegt ist,
i) eno-Gen, welches für Enolase codiert,
verstärkt ist oder gleichzeitig verstärkt sind.

9. Verfahren zur fermentativen Herstellung von L-Lysin gemäß Anspruch 5, wobei in den coryneformen Bakterien eines oder mehrere Gene, die aus der Gruppe gewählt werden, die aus folgenden Genen besteht:
a) dapA-Gen, welches für Dihydrodipicolinat-Synthase codiert,
b) lysC-Gen, welches für eine rückkopplungsresistente Aspartat-Kinase codiert,
c) gap-Gen, welches für Glycerinaldehyd-3-phosphat-Dehydrogenase codiert,
d) pyc-Gen, welches für Pyruvat-Carboxylase codiert,
e) tkt-Gen, welches für Transketolase codiert,
f) gnd-Gen, welches für Glucose-6-phosphat-Dehydrogenase codiert,
g) lysE-Gen, welches für den Lysinexport codiert,
h) als zwa1 bezeichnetes Gen, welches unter der Nummer DSM 13115 hinterlegt ist,
i) eno-Gen, welches für Enolase codiert,
überexprimiert wird oder gleichzeitig überexprimiert werden.

10. Verfahren zur fermentativen Herstellung von L-Threonin gemäß Anspruch 5, wobei in den coryneformen Bakterien eines oder mehrere Gene, die aus der Gruppe gewählt werden, die aus folgenden Genen besteht:
a) hom-Gen, welches für Homoserin-Dehydrogenase codiert oder hom^{dr}-Allel, welches für eine "rückkopplungsresistente" Homoserin-Dehydrogenase codiert,
b) gap-Gen, welches für Glycerinaldehyd-3-phosphat-Dehydrogenase codiert,
c) pyc-Gen, welches für Pyruvat-Carboxylase codiert,
d) mqo-Gen, welches für malat:chinon-Oxidoreductase codiert,
e) tkt-Gen, welches für Transketolase codiert,
f) gnd-Gen, welches für 6-Phosphogluconat-Dehydrogenase codiert,
g) thrE-Gen, welches für den Threoninexport codiert,
h) als zwa1 bezeichnetes Gen, welches unter der Nummer DSM 13115 hinterlegt ist,
i) eno-Gen, welches für Enolase codiert,
verstärkt ist oder gleichzeitig verstärkt sind.

11. Verfahren zur fermentativen Herstellung von L-Threonin gemäß Anspruch 5, wobei in den coryneformen Bakterien eines oder mehrere Gene, die aus der Gruppe gewählt werden, die aus folgenden Genen besteht:
a) hom-Gen, welches für Homoserin-Dehydrogenase codiert oder hom^{dr}-Allel, welches für eine "rückkopplungsresistente" Homoserin-Dehydrogenase codiert,
b) gap-Gen, welches für Glycerinaldehyd-3-phosphat-Dehydrogenase codiert,
c) pyc-Gen, welches für Pyruvat-Carboxylase codiert,
d) mqo-Gen, welches für malat:chinon-Oxidoreductase codiert,
e) tkt-Gen, welches für Transketolase codiert,
f) gnd-Gen, welches für 6-Phosphogluconat-Dehydrogenase codiert,
g) thrE-Gen, welches für den Threoninexport codiert,
h) als zwa1 bezeichnetes Gen, welches unter der Nummer DSM 13115 hinterlegt ist,
i) eno-Gen, welches für Enolase codiert,
überexprimiert wird oder gleichzeitig überexprimiert werden.

12. Verfahren zur fermentativen Herstellung von L-Lysin oder L-Threonin gemäß Anspruch 5, wobei Bakterien, in denen eines oder mehrere Gene, die aus der Gruppe gewählt werden, die aus folgenden Genen besteht:
a) pck-Gen, welches für Phosphoenolpyruvat-Carboxylase codiert,
b) pgi-Gen, welches für Glucose-6-phosphat-Isomerase codiert,
c) poxB-Gen, welches für Pyruvat-Oxidase codiert, oder
d) ein als zwa2 bezeichnetes Gen, welches unter der Nummer DSM 13113 hinterlegt ist,
abgeschwächt ist oder gleichzeitig abgeschwächt sind, fermentiert werden.

13. Verfahren nach Anspruch 5, wobei die Anzahl der Kopien des zwf-Gens gemäß Anspruch 1 durch Veränderung der Bakterien mittels Plasmid-Vektoren, die dieses Gen tragen, erhöht wird, um die Verstärkung zu erzielen.

14. Verfahren nach Anspruch 5 bis 11, wobei die Anzahl der Kopien der weiteren Gene oder Nucleotidsequenzen durch Veränderung der Bakterien mittels Plasmid-Vektoren, die diese Gene oder Nucleotidsequenzen tragen, erhöht wird, um die Verstärkung der Gene oder Nucleotidsequenzen zu erzielen.

15. Verfahren nach Anspruch 5, wobei Allele des zwf-Gens gemäß Anspruch 1, die aufgrund der Degenerationsneigung des genetischen Codes entstehen, verstärkt werden.

16. Verfahren nach Anspruch 5, wobei Allele des zwf-Gens gemäß Anspruch 1, die aufgrund von sense-Mutationen neutraler Art entstehen, verstärkt werden.

## Revendications

1. Gène isolé de Corynebacterium glutamicum (gène zwf), que l'on peut obtenir par PCR à partir de C. glutamicum ATCC13032 au moyen des amorces oligonucléotidiques suivantes :
zwf-sens (SEQ ID n° 10) :
5'-TCG ACG CGG TTC TGG AGC AG-3',
zwf-antisens (SEQ ID n° 11) :
5'-CTA AAT TAT GGC CTG CGC CAG-3',
codant pour un polypeptide ayant l'activité de la glucose-6-phosphate déshydrogénase, ledit polypeptide contenant la séquence d'acides aminés N-terminale (SEQ ID n° 9) Ser Thr Asn Thr Thr Pro Ser Ser Trp Thr Asn Pro Leu Arg Asp.

2. Bactéries coryneformes recombinantes productrices de L-lysine ou de L-thréonine, lesdites bactéries étant transformées par le gène selon la revendication 1, codant pour un polypeptide qui a l'activité de la glucose 6-phosphate déshydrogénase et qui contient la séquence d'acides aminés N-terminale (SEQ ID n° 9) Ser Thr Asn Thr Thr Pro Ser Ser Trp Thr Asn Pro Leu Arg Asp.

3. Bactéries coryneformes selon la revendication 2, dans lesquelles ledit gène est amplifié en augmentant le nombre de copies dudit gène.

4. Bactéries coryneformes selon la revendication 2, dans lesquelles ledit gène est lié à un promoteur.

5. Procédé pour la préparation de L-lysine ou de L-thréonine par fermentation de bactéries coryneformes, qui comprend la réalisation des étapes suivantes :
a) la fermentation desdites bactéries productrices d'acide aminé L, dans laquelle au moins le gène zwf codant pour la glucose-6-phosphate déshydrogénase selon la revendication 1 est amplifié,
b) la concentration dudit acide aminé L dans le milieu ou dans les cellules des bactéries, et
c) l'isolement dudit acide aminé L produit.

6. Procédé selon la revendication 5, dans lequel on utilise des bactéries coryneformes qui préparent de la L-thréonine ou de la L-lysine.

7. Procédé selon la revendication 6, dans lequel on utilise des bactéries du genre Corynebacterium qui préparent de la L-lysine.

8. Procédé pour la préparation de L-lysine par fermentation selon la revendication 5, dans lequel on amplifie simultanément dans les bactéries coryneformes un ou plusieurs gènes choisis dans le groupe constitué :
a) du gène dapA qui code pour la dihydrodipicolinate synthase,
b) du gène lysC qui code pour une aspartate kinase résistant à la rétroaction,
c) du gène gap qui code pour la glycérolaldéhyde 3-phosphate déshydrogénase,
d) du gène pyc qui code pour la pyruvate carboxylase,
e) du gène tkt qui code pour la transcétolase,
f) du gène gnd qui code pour la glucose 6-phosphate déshydrogénase,
g) du gène lysE qui code pour une protéine d'export de la lysine,
h) du gène dénommé zwa1 déposé sous la référence DSM 13115,
i) du gène eno qui code pour l'énolase.

9. Procédé pour la préparation de L-lysine par fermentation selon la revendication 5, dans lequel on surexprime simultanément dans les bactéries coryneformes un ou plusieurs gènes choisis dans le groupe constitué :
a) du gène dapA qui code pour la dihydrodipicolinate synthase,
b) du gène lysC qui code pour une aspartate kinase résistant à la rétroaction,
c) du gène gap qui code pour la glycérolaldéhyde-3-phosphate déshydrogénase,
d) du gène pyc qui code pour la pyruvate carboxylase,
e) du gène tkt qui code pour la transcétolase,
f) du gène gnd qui code pour la glucose 6-phosphate déshydrogénase,
g) du gène lysE qui code pour une protéine d'export de la lysine,
h) du gène dénommé zwa1 déposé sous la référence DSM 13115,
i) du gène eno qui code pour l'énolase.

10. Procédé pour la préparation de L-thréonine par fermentation selon la revendication 5, dans lequel on amplifie simultanément dans les bactéries coryneformes un ou plusieurs gènes choisis dans le groupe constitué :
a) du gène hom qui code pour l'homosérine déshydrogénase ou de l'allèle hom^{dr} qui code pour une homosérine déshydrogénase "résistant à la rétroaction",
b) du gène gap qui code pour la glycérolaldéhyde 3-phosphate déshydrogénase,
c) du gène pyc qui code pour la pyruvate carboxylase,
d) du gène mqo qui code pour la malate:quinone oxydoréductase,
e) du gène tkt qui code pour la transcétolase,
f) du gène gnd qui code pour la 6-phosphogluconate déshydrogénase,
g) du gène thrE qui code pour une protéine d'export de la thréonine,
h) du gène dénommé zwa1 déposé sous la référence DSM 13115,
i) du gène eno qui code pour l'énolase.

11. Procédé pour la préparation de L-thréonine par fermentation selon la revendication 5, dans lequel on surexprime simultanément dans les bactéries coryneformes un ou plusieurs gènes choisis dans le groupe constitué :
a) du gène hom qui code pour l'homosérine déshydrogénase ou de l'allèle hom^{dr} qui code pour une homosérine déshydrogénase "résistant à la rétroaction",
b) du gène gap qui code pour la glycérolaldéhyde 3-phosphate déshydrogénase,
c) du gène pyc qui code pour la pyruvate carboxylase,
d) du gène mqo qui code pour la malate:quinone oxydoréductase,
e) du gène tkt qui code pour la transcétolase,
f) du gène gnd qui code pour la 6-phosphogluconate déshydrogénase,
g) du gène thrE qui code pour une protéine d'export de la thréonine, du gène dénommé zwa1 déposé sous la référence DSM 13115,
i) du gène eno qui code pour l'énolase.

12. Procédé selon la revendication 5, dans lequel on fait fermenter pour la préparation de L-lysine ou de L-thréonine des bactéries dans lesquelles on a atténué simultanément un ou plusieurs gènes choisis dans le groupe constitué :
a) du gène pck qui code pour la phosphoénol pyruvate carboxykinase,
b) du gène pgi qui code pour la glucose 6-phosphate isomérase,
c) du gène poxB qui code pour la pyruvate oxydase ou
d) du gène dénommé zwa2 déposé sous la référence DSM 13113.

13. Procédé selon la revendication 5, dans lequel, pour obtenir l'amplification, on accroît le nombre de copies du gène zwf selon la revendication 1 en transformant les bactéries avec des vecteurs plasmidiques qui portent ce gène.

14. Procédé selon les revendications 5 à 11, dans lequel, pour obtenir l'amplification de gènes ou de séquences nucléotidiques supplémentaires, on accroît le nombre de copies desdits gènes ou desdites séquences nucléotidiques en transformant les bactéries avec des vecteurs plasmidiques qui portent lesdits gènes ou lesdites séquences nucléotidiques.

15. Procédé selon la revendication 5, dans lequel on amplifie les allèles du gène zwf selon la revendication 1, qui résultent de la dégénérescence du code génétique.

16. Procédé selon la revendication 5, dans lequel on amplifie les allèles du gène zwf selon la revendication 1, qui résultent de mutations sens à effet neutre.


### SEQUENCE PROTOCOL

<110> Degussa-Hüls AG
   National University of Ireland, Galway
   Forschungszentrum Jülich GmbH
<120> Process for the fermentative preparation of L-amino acids using the zwf gene.
<130> 990239BT
<140>
   <141>
<160> 6
<170> PatentIn Ver. 2.1
<210> 1
   <211> 2811
   <212> DNA
   <213> Corynebacterium glutamicum
<220>
   <221> CDS
   <222> (373)..(2022)
   <223> pgi
<400> 1
   aaaacccgag gggcgaaaat tccaccctaa cttttttggg atcccctttt tccggggaat 60
   taattggttt gggtttcaat gggaaaacgg gaaacaatgg gccaaaggtt caaaaacccc 120
   aaaagggggc cgggttcaaa ttcccaaaaa aaatggcaaa aaaggggggg ccaaaaccaa 180
   gttggccccc aaaccaccgg ggcaacggcc cacccacaaa ggggttgggt taaaggaagg 240
   acgcccaaag taagcccgga atggcccacg ttcgaaaaag caggccccaa ttaaacgcac 300
   cttaaatttg tcgtgtttcc cactttgaac actcttcgat gcgcttggcc acaaaagcaa 360
   gctaacctga ag atg tta ttt aac gac aat aaa gga gtt ttc atg gcg gac 411
                 Met Leu Phe Asn Asp Asn Lys Gly Val Phe Met Ala Asp
                 1               5                   10
   att tcg acc acc cag gtt tgg caa gac ctg acc gat cat tac tca aac    459
   Ile Ser Thr Thr Gln Val Trp Gln Asp Leu Thr Asp His Tyr Ser Asn
       15                  20                  25
   ttc cag gca acc act ctg cgt gaa ctt ttc aag gaa gaa aac cgc gcc    507
   Phe Gln Ala Thr Thr Leu Arg Glu Leu Phe Lys Glu Glu Asn Arg Ala
   30                  35                  40                  45
   gag aag tac acc ttc tcc gcg gct ggc ctc cac gtc gac ctg tcg aag    555
   Glu Lys Tyr Thr Phe Ser Ala Ala Gly Leu His Val Asp Leu Ser Lys
                   50                  55                  60
   aat ctg ctt gac gac gcc acc ctc acc aag ctc ctt gca ctg acc gaa    603
   Asn Leu Leu Asp Asp Ala Thr Leu Thr Lys Leu Leu Ala Leu Thr Glu
                   65                  70                  75
   gaa tct ggc ctt cgc gaa cgc att gac gcg atg ttt gcc ggt gaa cac     651
   Glu Ser Gly Leu Arg Glu Arg Ile Asp Ala Met Phe Ala Gly Glu His
           80                  85                  90
   ctc aac aac acc gaa gac cgc gct gtc ctc cac acc gcg ctg cgc ctt     699
   Leu Asn Asn Thr Glu Asp Arg Ala Val Leu His Thr Ala Leu Arg Leu
       95                  100                 105
   cct gcc gaa gct gat ctg tca gta gat ggc caa gat gtt gct gct gat     747
   Pro Ala Glu Ala Asp Leu Ser Val Asp Gly Gln Asp Val Ala Ala Asp
   110                 115                 120                 125
   gtc cac gaa gtt ttg gga cgc atg cgt gac ttc gct act gcg ctg cgc     795
   Val His Glu Val Leu Gly Arg Met Arg Asp Phe Ala Thr Ala Leu Arg
                   130                 135                 140
   tca ggc aac tgg ttg gga cac acc ggc cac acg atc aag aag atc gtc     843
   Ser Gly Asn Trp Leu Gly His Thr Gly His Thr Ile Lys Lys Ile Val
               145                 150                 155
   aac att ggt atc ggt ggc tct gac ctc gga cca gcc atg gct acg aag     891
   Asn Ile Gly Ile Gly Gly Ser Asp Leu Gly Pro Ala Met Ala Thr Lys
           160                 165                 170
   gct ctg cgt gca tac gcg acc gct ggt atc tca gca gaa ttc gtc tcc     939
   Ala Leu Arg Ala Tyr Ala Thr Ala Gly Ile Ser Ala Glu Phe Val Ser
       175                 180                 185
   aac gtc gac cca gca gac ctc gtt tct gtg ttg gaa gac ctc gat gca     987
   Asn Val Asp Pro Ala Asp Leu Val Ser Val Leu Glu Asp Leu Asp Ala
   190                 195                 200                 205
   gaa tcc aca ttg ttc gtg atc gct tcg aaa act ttc acc acc cag gag     1035
   Glu Ser Thr Leu Phe Val Ile Ala Ser Lys Thr Phe Thr Thr Gln Glu
                   210                 215                 220
   acg ctg tcc aac gct cgt gca gct cgt gct tgg ctg gta gag aag ctc     1083
   Thr Leu Ser Asn Ala Arg Ala Ala Arg Ala Trp Leu Val Glu Lys Leu
               225                 230                 235
   ggt gaa gag gct gtc gcg aag cac ttc gtc gca gtg tcc acc aat gct     1131
   Gly Glu Glu Ala Val Ala Lys His Phe Val Ala Val Ser Thr Asn Ala
           240                 245                 250
   gaa aag gtc gca gag ttc ggt atc gac acg gac aac atg ttc ggc ttc     1179
   Glu Lys Val Ala Glu Phe Gly Ile Asp Thr Asp Asn Met Phe Gly Phe
       255                 260                 265
   tgg gac tgg gtc gga ggt cgt tac tcc gtg gac tcc gca gtt ggt ctt     1227
   Trp Asp Trp Val Gly Gly Arg Tyr Ser Val Asp Ser Ala Val Gly Leu
   270                 275                 280                 285
   tcc ctc atg gca gtg atc ggc cct cgc gac ttc atg cgt ttc ctc ggt     1275
   Ser Leu Met Ala Val Ile Gly Pro Arg Asp Phe Met Arg Phe Leu Gly
                   290                 295                 300
   gga ttc cac gcg atg gat gaa cac ttc cgc acc acc aag ttc gaa gag     1323
   Gly Phe His Ala Met Asp Glu His Phe Arg Thr Thr Lys Phe Glu Glu
               305                 310                 315
   aac gtt cca atc ttg atg gct ctg ctc ggt gtc tgg tac tcc gat ttc     1371
   Asn Val Pro Ile Leu Met Ala Leu Leu Gly Val Trp Tyr Ser Asp Phe
           320                 325                 330
   tat ggt gca gaa acc cac gct gtc cta cct tat tcc gag gat ctc agc     1419
   Tyr Gly Ala Glu Thr His Ala Val Leu Pro Tyr Ser Glu Asp Leu Ser
       335                 340                 345
   cgt ttt gct gct tac ctc cag cag ctg acc atg gag acc aat ggc aag     1467
   Arg Phe Ala Ala Tyr Leu Gln Gln Leu Thr Met Glu Thr Asn Gly Lys
   350                 355                 360                 365
   tca gtc cac cgc gac ggc tcc cct gtt tcc act ggc act ggc gaa att     1515
   Ser Val His Arg Asp Gly Ser Pro Val Ser Thr Gly Thr Gly Glu Ile
                   370                 375                 380
   tac tgg ggt gag cct ggc aca aat ggc cag cac gct ttc ttc cag ctg     1563
   Tyr Trp Gly Glu Pro Gly Thr Asn Gly Gln His Ala Phe Phe Gln Leu
               385                 390                 395
   atc cac cag ggc act cgc ctt gtt cca gct gat ttc att ggt ttc gct     1611
   Ile His Gln Gly Thr Arg Leu Val Pro Ala Asp Phe Ile Gly Phe Ala
           400                 405                 410
   cgt cca aag cag gat ctt cct gcc ggt gag cgc acc atg cat gac ctt     1659
   Arg Pro Lys Gln Asp Leu Pro Ala Gly Glu Arg Thr Met His Asp Leu
       415                 420                 425
   ttg atg agc aac ttc ttc gca cag acc aag gtt ttg gct ttc ggt aag     1707
   Leu Met Ser Asn Phe Phe Ala Gln Thr Lys Val Leu Ala Phe Gly Lys
   430                 435                 440                 445
   aac gct gaa gag atc gct gcg gaa ggt gtc gca cct gag ctg gtc aac     1755
   Asn Ala Glu Glu Ile Ala Ala Glu Gly Val Ala Pro Glu Leu Val Asn
                   450                 455                 460
   cac aag gtc gtg cca ggt aat cgc cca acc acc acc att ttg gcg gag     1803
   His Lys Val Val Pro Gly Asn Arg Pro Thr Thr Thr Ile Leu Ala Glu
               465                 470                 475
   gaa ctt acc cct tct att ctc ggt gcg ttg atc gct ttg tac gaa cac     1851
   Glu Leu Thr Pro Ser Ile Leu Gly Ala Leu Ile Ala Leu Tyr Glu His
           480                 485                 490
   acc gtg atg gtt cag ggc gtg att tgg gac atc aac tcc ttc gac caa     1899
   Thr Val Met Val Gln Gly Val Ile Trp Asp Ile Asn Ser Phe Asp Gln
       495                 500                 505
   tgg ggt gtt gaa ctg ggc aaa cag cag gca aat gac ctc gct ccg gct     1947
   Trp Gly Val Glu Leu Gly Lys Gln Gln Ala Asn Asp Leu Ala Pro Ala
   510                 515                 520                 525
   gtc tct ggt gaa gag gat gtt gac tcg gga gat tct tcc act gat tca     1995
   Val Ser Gly Glu Glu Asp Val Asp Ser Gly Asp Ser Ser Thr Asp Ser
                   530                 535                 540
   ctg att aag tgg tac cgc gca aat agg tagtcgcttg cttatagggt        2042
   Leu Ile Lys Trp Tyr Arg Ala Asn Arg
               545                 550
   caggggcgtg aagaatcctc gcctcatagc actggccgct atcatcctga cctcgttcaa 2102
   tctgcgaaca gctattactg ctttagctcc gctggtttct gagattcggg atgatttagg 2162
   ggttagtgct tctcttattg gtgtgttggg catgatcccg actgctatgt tcgcggttgc 2222
   tgcgtttgcg cttccgtcgt tgaagaggaa gttcactact tcccaactgt tgatgtttgc 2282
   catgctgttg actgctgccg gtcagattat tcgtgtcgct ggacctgctt cgctgttgat 2342
   ggtcggtact gtgttcgcga tgtttgcgat cggagttacc aatgtgttgc ttccgattgc 2402
   tgttagggag tattttccgc gtcacgtcgg tggaatgtcg acaacttatc tggtgtcgtt 2462
   ccagattgtt caggcacttg ctccgacgct tgccgtgccg atttctcagt gggctacaca 2522
   tgtggggttg accggttgga gggtgtcgct cggttcgtgg gcgctgctgg ggttggttgc 2582
   ggcgatttcg tggattccgc tgttgagttt gcagggtgcc agggttgttg cggcgccgtc 2642
   gaaggtttct cttcctgtgt ggaagtcttc ggttggtgtg gggctcgggt tgatgtttgg 2702
   gtttacttcg tttgcgacgt atatcctcat gggttttatg ccgcagatgg taggtgatcc 2762
   aaagaattca aaaagcttct cgagagtact tctagagcgg ccgcgggcc             2811
<210> 2
   <211> 550
   <212> PRT
   <213> Corynebacterium glutamicum
<400> 2
   Met Leu Phe Asn Asp Asn Lys Gly Val Phe Met Ala Asp Ile Ser Thr
    1               5                  10                  15
   Thr Gln Val Trp Gln Asp Leu Thr Asp His Tyr Ser Asn Phe Gln Ala
               20                  25                  30
   Thr Thr Leu Arg Glu Leu Phe Lys Glu Glu Asn Arg Ala Glu Lys Tyr
           35                  40                  45
   Thr Phe Ser Ala Ala Gly Leu His Val Asp Leu Ser Lys Asn Leu Leu
       50                  55                  60
   Asp Asp Ala Thr Leu Thr Lys Leu Leu Ala Leu Thr Glu Glu Ser Gly
   65                  70                  75                  80
   Leu Arg Glu Arg Ile Asp Ala Met Phe Ala Gly Glu His Leu Asn Asn
                   85                  90                  95
   Thr Glu Asp Arg Ala Val Leu His Thr Ala Leu Arg Leu Pro Ala Glu
               100                 105                 110
   Ala Asp Leu Ser Val Asp Gly Gln Asp Val Ala Ala Asp Val His Glu
   115 120 125
   Val Leu Gly Arg Met Arg Asp Phe Ala Thr Ala Leu Arg Ser Gly Asn
   130 135 140
   Trp Leu Gly His Thr Gly His Thr Ile Lys Lys Ile Val Asn Ile Gly
   145 150 155 160
   Ile Gly Gly Ser Asp Leu Gly Pro Ala Met Ala Thr Lys Ala Leu Arg
   165 170 175
   Ala Tyr Ala Thr Ala Gly Ile Ser Ala Glu Phe Val Ser Asn Val Asp
   180 185 190
   Pro Ala Asp Leu Val Ser Val Leu Glu Asp Leu Asp Ala Glu Ser Thr
   195 200 205
   Leu Phe Val Ile Ala Ser Lys Thr Phe Thr Thr Gln Glu Thr Leu Ser
   210 215 220
   Asn Ala Arg Ala Ala Arg Ala Trp Leu Val Glu Lys Leu Gly Glu Glu
   225 230 235 240
   Ala Val Ala Lys His Phe Val Ala Val Ser Thr Asn Ala Glu Lys Val
   245 250 255
   Ala Glu Phe Gly Ile Asp Thr Asp Asn Met Phe Gly Phe Trp Asp Trp
   260 265 270
   Val Gly Gly Arg Tyr Ser Val Asp Ser Ala Val Gly Leu Ser Leu Met
   275 280 285
   Ala Val Ile Gly Pro Arg Asp Phe Met Arg Phe Leu Gly Gly Phe His
   290 295 300
   Ala Met Asp Glu His Phe Arg Thr Thr Lys Phe Glu Glu Asn Val Pro
   305 310 315 320
   Ile Leu Met Ala Leu Leu Gly Val Trp Tyr Ser Asp Phe Tyr Gly Ala
   325 330 335
   Glu Thr His Ala Val Leu Pro Tyr Ser Glu Asp Leu Ser Arg Phe Ala
   340 345 350
   Ala Tyr Leu Gln Gln Leu Thr Met Glu Thr Asn Gly Lys Ser Val His
   355 360 365
   Arg Asp Gly Ser Pro Val Ser Thr Gly Thr Gly Glu Ile Tyr Trp Gly
   370 375 380
   Glu Pro Gly Thr Asn Gly Gln His Ala Phe Phe Gln Leu Ile His Gln
   385 390 395 400
   Gly Thr Arg Leu Val Pro Ala Asp Phe Ile Gly Phe Ala Arg Pro Lys
   405 410 415
   Gln Asp Leu Pro Ala Gly Glu Arg Thr Met His Asp Leu Leu Met Ser
   420 425 430
   Asn Phe Phe Ala Gln Thr Lys Val Leu Ala Phe Gly Lys Asn Ala Glu
           435                 440                 445
   Glu Ile Ala Ala Glu Gly Val Ala Pro Glu Leu Val Asn His Lys Val
       450                 455                 460
   Val Pro Gly Asn Arg Pro Thr Thr Thr Ile Leu Ala Glu Glu Leu Thr
   465                 470                 475                 480
   Pro Ser Ile Leu Gly Ala Leu Ile Ala Leu Tyr Glu His Thr Val Met
                   485                 490                 495
   Val Gln Gly Val Ile Trp Asp Ile Asn Ser Phe Asp Gln Trp Gly Val
               500                 505                 510
   Glu Leu Gly Lys Gln Gln Ala Asn Asp Leu Ala Pro Ala Val Ser Gly
           515                 520                 525
   Glu Glu Asp Val Asp Ser Gly Asp Ser Ser Thr Asp Ser Leu Ile Lys
       530                 535                 540
   Trp Tyr Arg Ala Asn Arg
   545                 550
<210> 3
   <211> 462
   <212> DNA
   <213> Corynebacterium glutamicum
<400> 3
   atggagacca atggcaagtc agtccaccgc gacggctccc ctgtttccac tggcactggc 60
   gaaatttact ggggtgagcc tggcacaaat ggccagcacg ctttcttcca gctgatccac 120
   cagggcactc gccttgttcc agctgatttc attggtttcg ctcgtccaaa gcaggatctt 180
   cctgccggtg agcgcaccat gcatgacctt ttgatgagca acttcttcgc acagaccaag 240
   gttttggctt tcggtaagaa cgctgaagag atcgctgcgg aaggtgtcgc acctgagctg 300
   gtcaaccaca aggtcgtgcc aggtaatcgc ccaaccacca ccattttggc ggaggaactt 360
   accccttcta ttctcggtgc gttgatcgct ttgtacgaac acaccgtgat ggttcagggc 420
   gtgatttggg acatcaactc cttcgaccaa tggggcgtgg aa 462
<210> 4
   <211> 2160
   <212> DNA
   <213> Corynebacterium glutamicum
<220>
   <221> CDS
   <222> (327)..(2063)
   <223> poxB
<400> 4
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta 353
   Met Ala His Ser Tyr Ala Glu Gln Leu
   1 5
   att gac act ttg gaa gct caa ggt gtg aag cga att tat ggt ttg gtg 401
   Ile Asp Thr Leu Glu Ala Gln Gly Val Lys Arg Ile Tyr Gly Leu Val
   10 15 20 25
   ggt gac agc ctt aat ccg atc gtg gat gct gtc cgc caa tca gat att 449
   Gly Asp Ser Leu Asn Pro Ile Val Asp Ala Val Arg Gln Ser Asp Ile
   30 35 40
   gag tgg gtg cac gtt cga aat gag gaa gcg gcg gcg ttt gca gcc ggt 497
   Glu Trp Val His Val Arg Asn Glu Glu Ala Ala Ala Phe Ala Ala Gly
   45 50 55
   gcg gaa tcg ttg atc act ggg gag ctg gca gta tgt gct gct tct tgt 545
   Ala Glu Ser Leu Ile Thr Gly Glu Leu Ala Val Cys Ala Ala Ser Cys
   60 65 70
   ggt cct gga aac aca cac ctg att cag ggt ctt tat gat tcg cat cga 593
   Gly Pro Gly Asn Thr His Leu Ile Gln Gly Leu Tyr Asp Ser His Arg
   75 80 85
   aat ggt gcg aag gtg ttg gcc atc gct agc cat att ccg agt gcc cag 641
   Asn Gly Ala Lys Val Leu Ala Ile Ala Ser His Ile Pro Ser Ala Gln
   90 95 100 105
   att ggt tcg acg ttc ttc cag gaa acg cat ccg gag att ttg ttt aag 689
   Ile Gly Ser Thr Phe Phe Gln Glu Thr His Pro Glu Ile Leu Phe Lys
   110 115 120
   gaa tgc tct ggt tac tgc gag atg gtg aat ggt ggt gag cag ggt gaa 737
   Glu Cys Ser Gly Tyr Cys Glu Met Val Asn Gly Gly Glu Gln Gly Glu
   125 130 135
   cgc att ttg cat cac gcg att cag tcc acc atg gcg ggt aaa ggt gtg 785
   Arg Ile Leu His His Ala Ile Gln Ser Thr Met Ala Gly Lys Gly Val
   140 145 150
   tcg gtg gta gtg att cct ggt gat atc gct aag gaa gac gca ggt gac 833
   Ser Val Val Val Ile Pro Gly Asp Ile Ala Lys Glu Asp Ala Gly Asp
   155 160 165
   ggt act tat tcc aat tcc act att tct tct ggc act cct gtg gtg ttc 881
   Gly Thr Tyr Ser Asn Ser Thr Ile Ser Ser Gly Thr Pro Val Val Phe
   170 175 180 185
   ccg gat cct act gag gct gca gcg ctg gtg gag gcg att aac aac gct 929
   Pro Asp Pro Thr Glu Ala Ala Ala Leu Val Glu Ala Ile Asn Asn Ala
   190 195 200
   aag tct gtc act ttg ttc tgc ggt gcg ggc gtg aag aat gct cgc gcg 977
   Lys Ser Val Thr Leu Phe Cys Gly Ala Gly Val Lys Asn Ala Arg Ala
               205                 210                 215
   cag gtg ttg gag ttg gcg gag aag att aaa tca ccg atc ggg cat gcg    1025
   Gln Val Leu Glu Leu Ala Glu Lys Ile Lys Ser Pro Ile Gly His Ala
           220                 225                 230
   ctg ggt ggt aag cag tac atc cag cat gag aat ccg ttt gag gtc ggc    1073
   Leu Gly Gly Lys Gln Tyr Ile Gln His Glu Asn Pro Phe Glu Val Gly
       235                 240                 245
   atg tct ggc ctg ctt ggt tac ggc gcc tgc gtg gat gcg tcc aat gag    1121
   Met Ser Gly Leu Leu Gly Tyr Gly Ala Cys Val Asp Ala Ser Asn Glu
   250                 255                 260                 265
   gcg gat ctg ctg att cta ttg ggt acg gat ttc cct tat tct gat ttc    1169
   Ala Asp Leu Leu Ile Leu Leu Gly Thr Asp Phe Pro Tyr Ser Asp Phe
                   270                 275                 280
   ctt cct aaa gac aac gtt gcc cag gtg gat atc aac ggt gcg cac att    1217
   Leu Pro Lys Asp Asn Val Ala Gln Val Asp Ile Asn Gly Ala His Ile
               285                 290                 295
   ggt cga cgt acc acg gtg aag tat ccg gtg acc ggt gat gtt gct gca    1265
   Gly Arg Arg Thr Thr Val Lys Tyr Pro Val Thr Gly Asp Val Ala Ala
           300                 305                 310
   aca atc gaa aat att ttg cct cat gtg aag gaa aaa aca gat cgt tcc    1313
   Thr Ile Glu Asn Ile Leu Pro His Val Lys Glu Lys Thr Asp Arg Ser
       315                 320                 325
   ttc ctt gat cgg atg ctc aag gca cac gag cgt aag ttg agc tcg gtg    1361
   Phe Leu Asp Arg Met Leu Lys Ala His Glu Arg Lys Leu Ser Ser Val
   330                 335                 340                 345
   gta gag acg tac aca cat aac gtc gag aag cat gtg cct att cac cct    1409
   Val Glu Thr Tyr Thr His Asn Val Glu Lys His Val Pro Ile His Pro
                   350                 355                 360
   gaa tac gtt gcc tct att ttg aac gag ctg gcg gat aag gat gcg gtg    1457
   Glu Tyr Val Ala Ser Ile Leu Asn Glu Leu Ala Asp Lys Asp Ala Val
               365                 370                 375
   ttt act gtg gat acc ggc atg tgc aat gtg tgg cat gcg agg tac atc    1505
   Phe Thr Val Asp Thr Gly Met Cys Asn Val Trp His Ala Arg Tyr Ile
           380                 385                 390
   gag aat ccg gag gga acg cgc gac ttt gtg ggt tca ttc cgc cac ggc    1553
   Glu Asn Pro Glu Gly Thr Arg Asp Phe Val Gly Ser Phe Arg His Gly
       395                 400                 405
   acg atg gct aat gcg ttg cct cat gcg att ggt gcg caa agt gtt gat    1601
   Thr Met Ala Asn Ala Leu Pro His Ala Ile Gly Ala Gln Ser Val Asp
   410                 415                 420                 425
   cga aac cgc cag gtg atc gcg atg tgt ggc gat ggt ggt ttg ggc atg    1649
   Arg Asn Arg Gln Val Ile Ala Met Cys Gly Asp Gly Gly Leu Gly Met
                   430                 435                 440
   ctg ctg ggt gag ctt ctg acc gtt aag ctg cac caa ctt ccg ctg aag    1697
   Leu Leu Gly Glu Leu Leu Thr Val Lys Leu His Gln Leu Pro Leu Lys
               445                 450                 455
   gct gtg gtg ttt aac aac agt tct ttg ggc atg gtg aag ttg gag atg     1745
   Ala Val Val Phe Asn Asn Ser Ser Leu Gly Met Val Lys Leu Glu Met
           460                 465                 470
   ctc gtg gag gga cag cca gaa ttt ggt act gac cat gag gaa gtg aat     1793
   Leu Val Glu Gly Gln Pro Glu Phe Gly Thr Asp His Glu Glu Val Asn
       475                 480                 485
   ttc gca gag att gcg gcg gct gcg ggt atc aaa tcg gta cgc atc acc     1841
   Phe Ala Glu Ile Ala Ala Ala Ala Gly Ile Lys Ser Val Arg Ile Thr
   490                 495                 500                 505
   gat ccg aag aaa gtt cgc gag cag cta gct gag gca ttg gca tat cct     1889
   Asp Pro Lys Lys Val Arg Glu Gln Leu Ala Glu Ala Leu Ala Tyr Pro
                   510                 515                 520
   gga cct gta ctg atc gat atc gtc acg gat cct aat gcg ctg tcg atc     1937
   Gly Pro Val Leu Ile Asp Ile Val Thr Asp Pro Asn Ala Leu Ser Ile
               525                 530                 535
   cca cca acc atc acg tgg gaa cag gtc atg gga ttc agc aag gcg gcc     1985
   Pro Pro Thr Ile Thr Trp Glu Gln Val Met Gly Phe Ser Lys Ala Ala
           540                 545                 550
   acc cga acc gtc ttt ggt gga gga gta gga gcg atg atc gat ctg gcc     2033
   Thr Arg Thr Val Phe Gly Gly Gly Val Gly Ala Met Ile Asp Leu Ala
       555                 560                 565
   cgt tcg aac ata agg aat att cct act cca tgatgattga tacacctgct      2083
   Arg Ser Asn Ile Arg Asn Ile Pro Thr Pro
   570                 575
   gttctcattg accgcgagcg cttaactgcc aacatttcca ggatggcagc tcacgccggt 2143
   gcccatgaga ttgccct                                                2160
<210> 5
   <211> 579
   <212> PRT
   <213> Corynebacterium glutamicum
<400> 5
   Met Ala His Ser Tyr Ala Glu Gln Leu Ile Asp Thr Leu Glu Ala Gln
    1               5                  10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65                  70                  75                  80
   Ile Gln Gly Leu Tyr Asp Ser His Arg Asn Gly Ala Lys Val Leu Ala
                   85                  90                  95
   Ile Ala Ser His Ile Pro Ser Ala Gln Ile Gly Ser Thr Phe Phe Gln
               100                 105                 110
   Glu Thr His Pro Glu Ile Leu Phe Lys Glu Cys Ser Gly Tyr Cys Glu
           115                 120                 125
   Met Val Asn Gly Gly Glu Gln Gly Glu Arg Ile Leu His His Ala Ile
       130                 135                 140
   Gln Ser Thr Met Ala Gly Lys Gly Val Ser Val Val Val Ile Pro Gly
   145                 150                 155                 160
   Asp Ile Ala Lys Glu Asp Ala Gly Asp Gly Thr Tyr Ser Asn Ser Thr
                   165                 170                 175
   Ile Ser Ser Gly Thr Pro Val Val Phe Pro Asp Pro Thr Glu Ala Ala
               180                 185                 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
                   245                 250                 255
   Gly Ala Cys Val Asp Ala Ser Asn Glu Ala Asp Leu Leu Ile Leu Leu
               260                 265                 270
   Gly Thr Asp Phe Pro Tyr Ser Asp Phe Leu Pro Lys Asp Asn Val Ala
           275                 280                 285
   Gln Val Asp Ile Asn Gly Ala His Ile Gly Arg Arg Thr Thr Val Lys
       290                 295                 300
   Tyr Pro Val Thr Gly Asp Val Ala Ala Thr Ile Glu Asn Ile Leu Pro
   305                 310                 315                 320
   His Val Lys Glu Lys Thr Asp Arg Ser Phe Leu Asp Arg Met Leu Lys
                   325                 330                 335
   Ala His Glu Arg Lys Leu Ser Ser Val Val Glu Thr Tyr Thr His Asn
               340                 345                 350
   Val Glu Lys His Val Pro Ile His Pro Glu Tyr Val Ala Ser Ile Leu
           355                 360                 365
   Asn Glu Leu Ala Asp Lys Asp Ala Val Phe Thr Val Asp Thr Gly Met
       370                 375                 380
   Cys Asn Val Trp His Ala Arg Tyr Ile Glu Asn Pro Glu Gly Thr Arg
   385                 390                 395                 400
   Asp Phe Val Gly Ser Phe Arg His Gly Thr Met Ala Asn Ala Leu Pro
                   405                 410                 415
   His Ala Ile Gly Ala Gln Ser Val Asp Arg Asn Arg Gln Val Ile Ala
               420                 425                 430
   Met Cys Gly Asp Gly Gly Leu Gly Met Leu Leu Gly Glu Leu Leu Thr
           435                 440                 445
   Val Lys Leu His Gln Leu Pro Leu Lys Ala Val Val Phe Asn Asn Ser
       450                 455                 460
   Ser Leu Gly Met Val Lys Leu Glu Met Leu Val Glu Gly Gln Pro Glu
   465                 470                 475                 480
   Phe Gly Thr Asp His Glu Glu Val Asn Phe Ala Glu Ile Ala Ala Ala
                   485                 490                 495
   Ala Gly Ile Lys Ser Val Arg Ile Thr Asp Pro Lys Lys Val Arg Glu
               500                 505                 510
   Gln Leu Ala Glu Ala Leu Ala Tyr Pro Gly Pro Val Leu Ile Asp Ile
           515                 520                 525
   Val Thr Asp Pro Asn Ala Leu Ser Ile Pro Pro Thr Ile Thr Trp Glu
       530                 535                 540
   Gln Val Met Gly Phe Ser Lys Ala Ala Thr Arg Thr Val Phe Gly Gly
   545                 550                 555                 560
   Gly Val Gly Ala Met Ile Asp Leu Ala Arg Ser Asn Ile Arg Asn Ile
                   565                 570                 575
   Pro Thr Pro
<210> 6
   <211> 875
   <212> DNA
   <213> Corynebacterium glutamicum
<400> 6
   tgcgagatgg tgaatggtgg tgagcagggt gaacgcattt tgcatcacgc gattcagtcc 60
   accatggcgg gtaaaggtgt gtcggtggta gtgattcctg gtgatatcgc taaggaagac 120
   gcaggtgacg gtacttattc caattccact atttcttctg gcactcctgt ggtgttcccg 180
   gatcctactg aggctgcagc gctggtggag gcgattaaca acgctaagtc tgtcactttg 240
   ttctgcggtg cgggcgtgaa gaatgctcgc gcgcaggtgt tggagttggc ggagaagatt 300
   aaatcaccga tcgggcatgc gctgggtggt aagcagtaca tccagcatga gaatccgttt 360
   gaggtcggca tgtctggcct gcttggttac ggcgcctgcg tggatgcgtc caatgaggcg 420
   gatctgctga ttctattggg tacggatttc ccttattctg atttccttcc taaagacaac 480
   gttgcccagg tggatatcaa cggtgcgcac attggtcgac gtaccacggt gaagtatccg 540
   gtgaccggtg atgttgctgc aacaatcgaa aatattttgc ctcatgtgaa ggaaaaaaca 600
   gatcgttcct tccttgatcg gatgctcaag gcacacgagc gtaagttgag ctcggtggta 660
   gagacgtaca cacataacgt cgagaagcat gtgcctattc accctgaata cgttgcctct 720
   attttgaacg agctggcgga taaggatgcg gtgtttactg tggataccgg catgtgcaat 780
   gtgtggcatg cgaggtacat cgagaatccg gagggaacgc gcgactttgt gggttcattc 840
   cgccacggca cgatggctaa tgcgttgcct catgc                            875

### SEQUENCE LISTING

<110> Degussa AG
<120> Process for the fermentative preparation of L-amino acids with amplification of the zwf gene
<130> 990239BT
<140> PCT/EP00/06303
   <141> 2000-03-20
<160> 11
<170> PatentIn version 3.3
<210> 1
   <211> 2811
   <212> DNA
   <213> Corynebacterium glutamicum
<220>
   <221> CDS
   <222> (373)..(2022)
   <223> pgi
<400> 1
   aaaacccgag gggcgaaaat tccaccctaa cttttttggg atcccctttt tccggggaat 60
   taattggttt gggtttcaat gggaaaacgg gaaacaatgg gccaaaggtt caaaaacccc 120
   aaaagggggc cgggttcaaa ttcccaaaaa aaatggcaaa aaaggggggg ccaaaaccaa 180
   gttggccccc aaaccaccgg ggcaacggcc cacccacaaa ggggttgggt taaaggaagg 240
   acgcccaaag taagcccgga atggcccacg ttcgaaaaag caggccccaa ttaaacgcac 300
   cttaaatttg tcgtgtttcc cactttgaac actcttcgat gcgcttggcc acaaaagcaa 360
   gctaacctga ag atg tta ttt aac gac aat aaa gga gtt ttc atg gcg gac 411
   Met Leu Phe Asn Asp Asn Lys Gly Val Phe Met Ala Asp
   1 5 10
   att tcg acc acc cag gtt tgg caa gac ctg acc gat cat tac tca aac 459
   Ile Ser Thr Thr Gln Val Trp Gln Asp Leu Thr Asp His Tyr Ser Asn
   15 20 25
   ttc cag gca acc act ctg cgt gaa ctt ttc aag gaa gaa aac cgc gcc 507
   Phe Gln Ala Thr Thr Leu Arg Glu Leu Phe Lys Glu Glu Asn Arg Ala
   30 35 40 45
   gag aag tac acc ttc tcc gcg gct ggc ctc cac gtc gac ctg tcg aag 555
   Glu Lys Tyr Thr Phe Ser Ala Ala Gly Leu His Val Asp Leu Ser Lys
   50 55 60
   aat ctg ctt gac gac gcc acc ctc acc aag ctc ctt gca ctg acc gaa 603
   Asn Leu Leu Asp Asp Ala Thr Leu Thr Lys Leu Leu Ala Leu Thr Glu
   65 70 75
   gaa tct ggc ctt cgc gaa cgc att gac gcg atg ttt gcc ggt gaa cac 651
   Glu Ser Gly Leu Arg Glu Arg Ile Asp Ala Met Phe Ala Gly Glu His
   80 85 90
   ctc aac aac acc gaa gac cgc gct gtc ctc cac acc gcg ctg cgc ctt 699
   Leu Asn Asn Thr Glu Asp Arg Ala Val Leu His Thr Ala Leu Arg Leu
   95 100 105
   cct gcc gaa gct gat ctg tca gta gat ggc caa gat gtt gct gct gat 747
   Pro Ala Glu Ala Asp Leu Ser Val Asp Gly Gln Asp Val Ala Ala Asp
   110 115 120 125
   gtc cac gaa gtt ttg gga cgc atg cgt gac ttc gct act gcg ctg cgc 795
   Val His Glu Val Leu Gly Arg Met Arg Asp Phe Ala Thr Ala Leu Arg
   130 135 140
   tca ggc aac tgg ttg gga cac acc ggc cac acg atc aag aag atc gtc 843
   Ser Gly Asn Trp Leu Gly His Thr Gly His Thr Ile Lys Lys Ile Val
   145 150 155
   aac att ggt atc ggt ggc tct gac ctc gga cca gcc atg gct acg aag 891
   Asn Ile Gly Ile Gly Gly Ser Asp Leu Gly Pro Ala Met Ala Thr Lys
   160 165 170
   gct ctg cgt gca tac gcg acc gct ggt atc tca gca gaa ttc gtc tcc 939
   Ala Leu Arg Ala Tyr Ala Thr Ala Gly Ile Ser Ala Glu Phe Val Ser
   175 180 185
   aac gtc gac cca gca gac ctc gtt tct gtg ttg gaa gac ctc gat gca 987
   Asn Val Asp Pro Ala Asp Leu Val Ser Val Leu Glu Asp Leu Asp Ala
   190 195 200 205
   gaa tcc aca ttg ttc gtg atc gct tcg aaa act ttc acc acc cag gag 1035
   Glu Ser Thr Leu Phe Val Ile Ala Ser Lys Thr Phe Thr Thr Gln Glu
   210 215 220
   acg ctg tcc aac gct cgt gca gct cgt gct tgg ctg gta gag aag ctc 1083
   Thr Leu Ser Asn Ala Arg Ala Ala Arg Ala Trp Leu Val Glu Lys Leu
   225 230 235
   ggt gaa gag gct gtc gcg aag cac ttc gtc gca gtg tcc acc aat gct 1131
   Gly Glu Glu Ala Val Ala Lys His Phe Val Ala Val Ser Thr Asn Ala
   240 245 250
   gaa aag gtc gca gag ttc ggt atc gac acg gac aac atg ttc ggc ttc 1179
   Glu Lys Val Ala Glu Phe Gly Ile Asp Thr Asp Asn Met Phe Gly Phe
   255 260 265
   tgg gac tgg gtc gga ggt cgt tac tcc gtg gac tcc gca gtt ggt ctt 1227
   Trp Asp Trp Val Gly Gly Arg Tyr Ser Val Asp Ser Ala Val Gly Leu
   270 275 280 285
   tcc ctc atg gca gtg atc ggc cct cgc gac ttc atg cgt ttc ctc ggt 1275
   Ser Leu Met Ala Val Ile Gly Pro Arg Asp Phe Met Arg Phe Leu Gly
   290 295 300
   gga ttc cac gcg atg gat gaa cac ttc cgc acc acc aag ttc gaa gag 1323
   Gly Phe His Ala Met Asp Glu His Phe Arg Thr Thr Lys Phe Glu Glu
   305 310 315
   aac gtt cca atc ttg atg gct ctg ctc ggt gtc tgg tac tcc gat ttc 1371
   Asn Val Pro Ile Leu Met Ala Leu Leu Gly Val Trp Tyr Ser Asp Phe
   320 325 330
   tat ggt gca gaa acc cac gct gtc cta cct tat tcc gag gat ctc agc 1419
   Tyr Gly Ala Glu Thr His Ala Val Leu Pro Tyr Ser Glu Asp Leu Ser
   335 340 345
   cgt ttt gct gct tac ctc cag cag ctg acc atg gag acc aat ggc aag 1467
   Arg Phe Ala Ala Tyr Leu Gln Gln Leu Thr Met Glu Thr Asn Gly Lys
   350 355 360 365
   tca gtc cac cgc gac ggc tcc cct gtt tcc act ggc act ggc gaa att 1515
   Ser Val His Arg Asp Gly Ser Pro Val Ser Thr Gly Thr Gly Glu Ile
   370 375 380
   tac tgg ggt gag cct ggc aca aat ggc cag cac gct ttc ttc cag ctg 1563
   Tyr Trp Gly Glu Pro Gly Thr Asn Gly Gln His Ala Phe Phe Gln Leu
   385 390 395
   atc cac cag ggc act cgc ctt gtt cca gct gat ttc att ggt ttc gct 1611
   Ile His Gln Gly Thr Arg Leu Val Pro Ala Asp Phe Ile Gly Phe Ala
   400 405 410
   cgt cca aag cag gat ctt cct gcc ggt gag cgc acc atg cat gac ctt 1659
   Arg Pro Lys Gln Asp Leu Pro Ala Gly Glu Arg Thr Met His Asp Leu
   415 420 425
   ttg atg agc aac ttc ttc gca cag acc aag gtt ttg gct ttc ggt aag 1707
   Leu Met Ser Asn Phe Phe Ala Gln Thr Lys Val Leu Ala Phe Gly Lys
   430 435 440 445
   aac gct gaa gag atc gct gcg gaa ggt gtc gca cct gag ctg gtc aac 1755
   Asn Ala Glu Glu Ile Ala Ala Glu Gly Val Ala Pro Glu Leu Val Asn
   450 455 460
   cac aag gtc gtg cca ggt aat cgc cca acc acc acc att ttg gcg gag 1803
   His Lys Val Val Pro Gly Asn Arg Pro Thr Thr Thr Ile Leu Ala Glu
   465 470 475
   gaa ctt acc cct tct att ctc ggt gcg ttg atc gct ttg tac gaa cac 1851
   Glu Leu Thr Pro Ser Ile Leu Gly Ala Leu Ile Ala Leu Tyr Glu His
   480 485 490
   acc gtg atg gtt cag ggc gtg att tgg gac atc aac tcc ttc gac caa 1899
   Thr Val Met Val Gln Gly Val Ile Trp Asp Ile Asn Ser Phe Asp Gln
   495 500 505
   tgg ggt gtt gaa ctg ggc aaa cag cag gca aat gac ctc gct ccg gct 1947
   Trp Gly Val Glu Leu Gly Lys Gln Gln Ala Asn Asp Leu Ala Pro Ala
   510 515 520 525
   gtc tct ggt gaa gag gat gtt gac tcg gga gat tct tcc act gat tca 1995
   Val Ser Gly Glu Glu Asp Val Asp Ser Gly Asp Ser Ser Thr Asp Ser
   530 535 540
   ctg att aag tgg tac cgc gca aat agg tagtcgcttg cttatagggt 2042
   Leu Ile Lys Trp Tyr Arg Ala Asn Arg
   545 550
   caggggcgtg aagaatcctc gcctcatagc actggccgct atcatcctga cctcgttcaa 2102
   tctgcgaaca gctattactg ctttagctcc gctggtttct gagattcggg atgatttagg 2162
   ggttagtgct tctcttattg gtgtgttggg catgatcccg actgctatgt tcgcggttgc 2222
   tgcgtttgcg cttccgtcgt tgaagaggaa gttcactact tcccaactgt tgatgtttgc 2282
   catgctgttg actgctgccg gtcagattat tcgtgtcgct ggacctgctt cgctgttgat 2342
   ggtcggtact gtgttcgcga tgtttgcgat cggagttacc aatgtgttgc ttccgattgc 2402
   tgttagggag tattttccgc gtcacgtcgg tggaatgtcg acaacttatc tggtgtcgtt 2462
   ccagattgtt caggcacttg ctccgacgct tgccgtgccg atttctcagt gggctacaca 2522
   tgtggggttg accggttgga gggtgtcgct cggttcgtgg gcgctgctgg ggttggttgc 2582
   ggcgatttcg tggattccgc tgttgagttt gcagggtgcc agggttgttg cggcgccgtc 2642
   gaaggtttct cttcctgtgt ggaagtcttc ggttggtgtg gggctcgggt tgatgtttgg 2702
   gtttacttcg tttgcgacgt atatcctcat gggttttatg ccgcagatgg taggtgatcc 2762
   aaagaattca aaaagcttct cgagagtact tctagagcgg ccgcgggcc 2811
<210> 2
   <211> 550
   <212> PRT
   <213> Corynebacterium glutamicum
<400> 2
   Met Leu Phe Asn Asp Asn Lys Gly Val Phe Met Ala Asp Ile Ser Thr
   1 5 10 15
   Thr Gln Val Trp Gln Asp Leu Thr Asp His Tyr Ser Asn Phe Gln Ala
   20 25 30
   Thr Thr Leu Arg Glu Leu Phe Lys Glu Glu Asn Arg Ala Glu Lys Tyr
   35 40 45
   Thr Phe Ser Ala Ala Gly Leu His Val Asp Leu Ser Lys Asn Leu Leu
   50 55 60
   Asp Asp Ala Thr Leu Thr Lys Leu Leu Ala Leu Thr Glu Glu Ser Gly
   65 70 75 80
   Leu Arg Glu Arg Ile Asp Ala Met Phe Ala Gly Glu His Leu Asn Asn
   85 90 95
   Thr Glu Asp Arg Ala Val Leu His Thr Ala Leu Arg Leu Pro Ala Glu
   100 105 110
   Ala Asp Leu Ser Val Asp Gly Gln Asp Val Ala Ala Asp Val His Glu
   115 120 125
   Val Leu Gly Arg Met Arg Asp Phe Ala Thr Ala Leu Arg Ser Gly Asn
   130 135 140
   Trp Leu Gly His Thr Gly His Thr Ile Lys Lys Ile Val Asn Ile Gly
   145 150 155 160
   Ile Gly Gly Ser Asp Leu Gly Pro Ala Met Ala Thr Lys Ala Leu Arg
   165 170 175
   Ala Tyr Ala Thr Ala Gly Ile Ser Ala Glu Phe Val Ser Asn Val Asp
   180 185 190
   Pro Ala Asp Leu Val Ser Val Leu Glu Asp Leu Asp Ala Glu Ser Thr
   195 200 205
   Leu Phe Val Ile Ala Ser Lys Thr Phe Thr Thr Gln Glu Thr Leu Ser
   210 215 220
   Asn Ala Arg Ala Ala Arg Ala Trp Leu Val Glu Lys Leu Gly Glu Glu
   225 230 235 240
   Ala Val Ala Lys His Phe Val Ala Val Ser Thr Asn Ala Glu Lys Val
   245 250 255
   Ala Glu Phe Gly Ile Asp Thr Asp Asn Met Phe Gly Phe Trp Asp Trp
   260 265 270
   Val Gly Gly Arg Tyr Ser Val Asp Ser Ala Val Gly Leu Ser Leu Met
   275 280 285
   Ala Val Ile Gly Pro Arg Asp Phe Met Arg Phe Leu Gly Gly Phe His
   290 295 300
   Ala Met Asp Glu His Phe Arg Thr Thr Lys Phe Glu Glu Asn Val Pro
   305 310 315 320
   Ile Leu Met Ala Leu Leu Gly Val Trp Tyr Ser Asp Phe Tyr Gly Ala
   325 330 335
   Glu Thr His Ala Val Leu Pro Tyr Ser Glu Asp Leu Ser Arg Phe Ala
   340 345 350
   Ala Tyr Leu Gln Gln Leu Thr Met Glu Thr Asn Gly Lys Ser Val His
   355 360 365
   Arg Asp Gly Ser Pro Val Ser Thr Gly Thr Gly Glu Ile Tyr Trp Gly
   370 375 380
   Glu Pro Gly Thr Asn Gly Gln His Ala Phe Phe Gln Leu Ile His Gln
   385 390 395 400
   Gly Thr Arg Leu Val Pro Ala Asp Phe Ile Gly Phe Ala Arg Pro Lys
   405 410 415
   Gln Asp Leu Pro Ala Gly Glu Arg Thr Met His Asp Leu Leu Met Ser
   420 425 430
   Asn Phe Phe Ala Gln Thr Lys Val Leu Ala Phe Gly Lys Asn Ala Glu
   435 440 445
   Glu Ile Ala Ala Glu Gly Val Ala Pro Glu Leu Val Asn His Lys Val
   450 455 460
   Val Pro Gly Asn Arg Pro Thr Thr Thr Ile Leu Ala Glu Glu Leu Thr
   465 470 475 480
   Pro Ser Ile Leu Gly Ala Leu Ile Ala Leu Tyr Glu His Thr Val Met
   485 490 495
   Val Gln Gly Val Ile Trp Asp Ile Asn Ser Phe Asp Gln Trp Gly Val
   500 505 510
   Glu Leu Gly Lys Gln Gln Ala Asn Asp Leu Ala Pro Ala Val Ser Gly
   515 520 525
   Glu Glu Asp Val Asp Ser Gly Asp Ser Ser Thr Asp Ser Leu Ile Lys
   530 535 540
   Trp Tyr Arg Ala Asn Arg
   545 550
<210> 3
   <211> 462
   <212> DNA
   <213> Corynebacterium glutamicum
<400> 3
   atggagacca atggcaagtc agtccaccgc gacggctccc ctgtttccac tggcactggc 60
   gaaatttact ggggtgagcc tggcacaaat ggccagcacg ctttcttcca gctgatccac 120
   cagggcactc gccttgttcc agctgatttc attggtttcg ctcgtccaaa gcaggatctt 180
   cctgccggtg agcgcaccat gcatgacctt ttgatgagca acttcttcgc acagaccaag 240
   gttttggctt tcggtaagaa cgctgaagag atcgctgcgg aaggtgtcgc acctgagctg 300
   gtcaaccaca aggtcgtgcc aggtaatcgc ccaaccacca ccattttggc ggaggaactt 360
   accccttcta ttctcggtgc gttgatcgct ttgtacgaac acaccgtgat ggttcagggc 420
   gtgatttggg acatcaactc cttcgaccaa tggggcgtgg aa 462
<210> 4
   <211> 2160
   <212> DNA
   <213> Corynebacterium glutamicum
<220>
   <221> CDS
   <222> (327)..(2063)
   <223> poxB
<400> 4
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta 353
   Met Ala His Ser Tyr Ala Glu Gln Leu
   1 5
   att gac act ttg gaa gct caa ggt gtg aag cga att tat ggt ttg gtg 401
   Ile Asp Thr Leu Glu Ala Gln Gly Val Lys Arg Ile Tyr Gly Leu Val
   10 15 20 25
   ggt gac agc ctt aat ccg atc gtg gat gct gtc cgc caa tca gat att 449
   Gly Asp Ser Leu Asn Pro Ile Val Asp Ala Val Arg Gln Ser Asp Ile
   30 35 40
   gag tgg gtg cac gtt cga aat gag gaa gcg gcg gcg ttt gca gcc ggt 497
   Glu Trp Val His Val Arg Asn Glu Glu Ala Ala Ala Phe Ala Ala Gly
   45 50 55
   gcg gaa tcg ttg atc act ggg gag ctg gca gta tgt gct gct tct tgt 545
   Ala Glu Ser Leu Ile Thr Gly Glu Leu Ala Val Cys Ala Ala Ser Cys
   60 65 70
   ggt cct gga aac aca cac ctg att cag ggt ctt tat gat tcg cat cga 593
   Gly Pro Gly Asn Thr His Leu Ile Gln Gly Leu Tyr Asp Ser His Arg
   75 80 85
   aat ggt gcg aag gtg ttg gcc atc gct agc cat att ccg agt gcc cag 641
   Asn Gly Ala Lys Val Leu Ala Ile Ala Ser His Ile Pro Ser Ala Gln
   90 95 100 105
   att ggt tcg acg ttc ttc cag gaa acg cat ccg gag att ttg ttt aag 689
   Ile Gly Ser Thr Phe Phe Gln Glu Thr His Pro Glu Ile Leu Phe Lys
   110 115 120
   gaa tgc tct ggt tac tgc gag atg gtg aat ggt ggt gag cag ggt gaa 737
   Glu Cys Ser Gly Tyr Cys Glu Met Val Asn Gly Gly Glu Gln Gly Glu
   125 130 135
   cgc att ttg cat cac gcg att cag tcc acc atg gcg ggt aaa ggt gtg 785
   Arg Ile Leu His His Ala Ile Gln Ser Thr Met Ala Gly Lys Gly Val
   140 145 150
   tcg gtg gta gtg att cct ggt gat atc gct aag gaa gac gca ggt gac 833
   Ser Val Val Val Ile Pro Gly Asp Ile Ala Lys Glu Asp Ala Gly Asp
   155 160 165
   ggt act tat tcc aat tcc act att tct tct ggc act cct gtg gtg ttc 881
   Gly Thr Tyr Ser Asn Ser Thr Ile Ser Ser Gly Thr Pro Val Val Phe
   170 175 180 185
   ccg gat cct act gag gct gca gcg ctg gtg gag gcg att aac aac gct 929
   Pro Asp Pro Thr Glu Ala Ala Ala Leu Val Glu Ala Ile Asn Asn Ala
   190 195 200
   aag tct gtc act ttg ttc tgc ggt gcg ggc gtg aag aat gct cgc gcg 977
   Lys Ser Val Thr Leu Phe Cys Gly Ala Gly Val Lys Asn Ala Arg Ala
   205 210 215
   cag gtg ttg gag ttg gcg gag aag att aaa tca ccg atc ggg cat gcg 1025
   Gln Val Leu Glu Leu Ala Glu Lys Ile Lys Ser Pro Ile Gly His Ala
   220 225 230
   ctg ggt ggt aag cag tac atc cag cat gag aat ccg ttt gag gtc ggc 1073
   Leu Gly Gly Lys Gln Tyr Ile Gln His Glu Asn Pro Phe Glu Val Gly
   235 240 245
   atg tct ggc ctg ctt ggt tac ggc gcc tgc gtg gat gcg tcc aat gag 1121
   Met Ser Gly Leu Leu Gly Tyr Gly Ala Cys Val Asp Ala Ser Asn Glu
   250 255 260 265
   gcg gat ctg ctg att cta ttg ggt acg gat ttc cct tat tct gat ttc 1169
   Ala Asp Leu Leu Ile Leu Leu Gly Thr Asp Phe Pro Tyr Ser Asp Phe
   270 275 280
   ctt cct aaa gac aac gtt gcc cag gtg gat atc aac ggt gcg cac att 1217
   Leu Pro Lys Asp Asn Val Ala Gln Val Asp Ile Asn Gly Ala His Ile
   285 290 295
   ggt cga cgt acc acg gtg aag tat ccg gtg acc ggt gat gtt gct gca 1265
   Gly Arg Arg Thr Thr Val Lys Tyr Pro Val Thr Gly Asp Val Ala Ala
   300 305 310
   aca atc gaa aat att ttg cct cat gtg aag gaa aaa aca gat cgt tcc 1313
   Thr Ile Glu Asn Ile Leu Pro His Val Lys Glu Lys Thr Asp Arg Ser
   315 320 325
   ttc ctt gat cgg atg ctc aag gca cac gag cgt aag ttg agc tcg gtg 1361
   Phe Leu Asp Arg Met Leu Lys Ala His Glu Arg Lys Leu Ser Ser Val
   330 335 340 345
   gta gag acg tac aca cat aac gtc gag aag cat gtg cct att cac cct 1409
   Val Glu Thr Tyr Thr His Asn Val Glu Lys His Val Pro Ile His Pro
   350 355 360
   gaa tac gtt gcc tct att ttg aac gag ctg gcg gat aag gat gcg gtg 1457
   Glu Tyr Val Ala Ser Ile Leu Asn Glu Leu Ala Asp Lys Asp Ala Val
   365 370 375
   ttt act gtg gat acc ggc atg tgc aat gtg tgg cat gcg agg tac atc 1505
   Phe Thr Val Asp Thr Gly Met Cys Asn Val Trp His Ala Arg Tyr Ile
   380 385 390
   gag aat ccg gag gga acg cgc gac ttt gtg ggt tca ttc cgc cac ggc 1553
   Glu Asn Pro Glu Gly Thr Arg Asp Phe Val Gly Ser Phe Arg His Gly
   395 400 405
   acg atg gct aat gcg ttg cct cat gcg att ggt gcg caa agt gtt gat 1601
   Thr Met Ala Asn Ala Leu Pro His Ala Ile Gly Ala Gln Ser Val Asp
   410 415 420 425
   cga aac cgc cag gtg atc gcg atg tgt ggc gat ggt ggt ttg ggc atg 1649
   Arg Asn Arg Gln Val Ile Ala Met Cys Gly Asp Gly Gly Leu Gly Met
   430 435 440
   ctg ctg ggt gag ctt ctg acc gtt aag ctg cac caa ctt ccg ctg aag 1697
   Leu Leu Gly Glu Leu Leu Thr Val Lys Leu His Gln Leu Pro Leu Lys
   445 450 455
   gct gtg gtg ttt aac aac agt tct ttg ggc atg gtg aag ttg gag atg 1745
   Ala Val Val Phe Asn Asn Ser Ser Leu Gly Met Val Lys Leu Glu Met
   460 465 470
   ctc gtg gag gga cag cca gaa ttt ggt act gac cat gag gaa gtg aat 1793
   Leu Val Glu Gly Gln Pro Glu Phe Gly Thr Asp His Glu Glu Val Asn
   475 480 485
   ttc gca gag att gcg gcg gct gcg ggt atc aaa tcg gta cgc atc acc 1841
   Phe Ala Glu Ile Ala Ala Ala Ala Gly Ile Lys Ser Val Arg Ile Thr
   490 495 500 505
   gat ccg aag aaa gtt cgc gag cag cta gct gag gca ttg gca tat cct 1889
   Asp Pro Lys Lys Val Arg Glu Gln Leu Ala Glu Ala Leu Ala Tyr Pro
   510 515 520
   gga cct gta ctg atc gat atc gtc acg gat cct aat gcg ctg tcg atc 1937
   Gly Pro Val Leu Ile Asp Ile Val Thr Asp Pro Asn Ala Leu Ser Ile
   525 530 535
   cca cca acc atc acg tgg gaa cag gtc atg gga ttc agc aag gcg gcc 1985
   Pro Pro Thr Ile Thr Trp Glu Gln Val Met Gly Phe Ser Lys Ala Ala
   540 545 550
   acc cga acc gtc ttt ggt gga gga gta gga gcg atg atc gat ctg gcc 2033
   Thr Arg Thr Val Phe Gly Gly Gly Val Gly Ala Met Ile Asp Leu Ala
   555 560 565
   cgt tcg aac ata agg aat att cct act cca tgatgattga tacacctgct 2083
   Arg Ser Asn Ile Arg Asn Ile Pro Thr Pro
   570 575
   gttctcattg accgcgagcg cttaactgcc aacatttcca ggatggcagc tcacgccggt 2143
   gcccatgaga ttgccct 2160
<210> 5
   <211> 579
   <212> PRT
   <213> Corynebacterium glutamicum
<400> 5
   Met Ala His Ser Tyr Ala Glu Gln Leu Ile Asp Thr Leu Glu Ala Gln
   1 5 10 15
   Gly Val Lys Arg Ile Tyr Gly Leu Val Gly Asp Ser Leu Asn Pro Ile
   20 25 30
   Val Asp Ala Val Arg Gln Ser Asp Ile Glu Trp Val His Val Arg Asn
   35 40 45
   Glu Glu Ala Ala Ala Phe Ala Ala Gly Ala Glu Ser Leu Ile Thr Gly
   50 55 60
   Glu Leu Ala Val Cys Ala Ala Ser Cys Gly Pro Gly Asn Thr His Leu
   65 70 75 80
   Ile Gln Gly Leu Tyr Asp Ser His Arg Asn Gly Ala Lys Val Leu Ala
   85 90 95
   Ile Ala Ser His Ile Pro Ser Ala Gln Ile Gly Ser Thr Phe Phe Gln
   100 105 110
   Glu Thr His Pro Glu Ile Leu Phe Lys Glu Cys Ser Gly Tyr Cys Glu
   115 120 125
   Met Val Asn Gly Gly Glu Gln Gly Glu Arg Ile Leu His His Ala Ile
   130 135 140
   Gln Ser Thr Met Ala Gly Lys Gly Val Ser Val Val Val Ile Pro Gly
   145 150 155 160
   Asp Ile Ala Lys Glu Asp Ala Gly Asp Gly Thr Tyr Ser Asn Ser Thr
   165 170 175
   Ile Ser Ser Gly Thr Pro Val Val Phe Pro Asp Pro Thr Glu Ala Ala
   180 185 190
   Ala Leu Val Glu Ala Ile Asn Asn Ala Lys Ser Val Thr Leu Phe Cys
   195 200 205
   Gly Ala Gly Val Lys Asn Ala Arg Ala Gln Val Leu Glu Leu Ala Glu
   210 215 220
   Lys Ile Lys Ser Pro Ile Gly His Ala Leu Gly Gly Lys Gln Tyr Ile
   225 230 235 240
   Gln His Glu Asn Pro Phe Glu Val Gly Met Ser Gly Leu Leu Gly Tyr
   245 250 255
   Gly Ala Cys Val Asp Ala Ser Asn Glu Ala Asp Leu Leu Ile Leu Leu
   260 265 270
   Gly Thr Asp Phe Pro Tyr Ser Asp Phe Leu Pro Lys Asp Asn Val Ala
   275 280 285
   Gln Val Asp Ile Asn Gly Ala His Ile Gly Arg Arg Thr Thr Val Lys
   290 295 300
   Tyr Pro Val Thr Gly Asp Val Ala Ala Thr Ile Glu Asn Ile Leu Pro
   305 310 315 320
   His Val Lys Glu Lys Thr Asp Arg Ser Phe Leu Asp Arg Met Leu Lys
   325 330 335
   Ala His Glu Arg Lys Leu Ser Ser Val Val Glu Thr Tyr Thr His Asn
   340 345 350
   Val Glu Lys His Val Pro Ile His Pro Glu Tyr Val Ala Ser Ile Leu
   355 360 365
   Asn Glu Leu Ala Asp Lys Asp Ala Val Phe Thr Val Asp Thr Gly Met
   370 375 380
   Cys Asn Val Trp His Ala Arg Tyr Ile Glu Asn Pro Glu Gly Thr Arg
   385 390 395 400
   Asp Phe Val Gly Ser Phe Arg His Gly Thr Met Ala Asn Ala Leu Pro
   405 410 415
   His Ala Ile Gly Ala Gln Ser Val Asp Arg Asn Arg Gln Val Ile Ala
   420 425 430
   Met Cys Gly Asp Gly Gly Leu Gly Met Leu Leu Gly Glu Leu Leu Thr
   435 440 445
   Val Lys Leu His Gln Leu Pro Leu Lys Ala Val Val Phe Asn Asn Ser
   450 455 460
   Ser Leu Gly Met Val Lys Leu Glu Met Leu Val Glu Gly Gln Pro Glu
   465 470 475 480
   Phe Gly Thr Asp His Glu Glu Val Asn Phe Ala Glu Ile Ala Ala Ala
   485 490 495
   Ala Gly Ile Lys Ser Val Arg Ile Thr Asp Pro Lys Lys Val Arg Glu
   500 505 510
   Gln Leu Ala Glu Ala Leu Ala Tyr Pro Gly Pro Val Leu Ile Asp Ile
   515 520 525
   Val Thr Asp Pro Asn Ala Leu Ser Ile Pro Pro Thr Ile Thr Trp Glu
   530 535 540
   Gln Val Met Gly Phe Ser Lys Ala Ala Thr Arg Thr Val Phe Gly Gly
   545 550 555 560
   Gly Val Gly Ala Met Ile Asp Leu Ala Arg Ser Asn Ile Arg Asn Ile
   565 570 575
   Pro Thr Pro
<210> 6
   <211> 875
   <212> DNA
   <213> Corynebacterium glutamicum
<400> 6
   tgcgagatgg tgaatggtgg tgagcagggt gaacgcattt tgcatcacgc gattcagtcc 60
   accatggcgg gtaaaggtgt gtcggtggta gtgattcctg gtgatatcgc taaggaagac 120
   gcaggtgacg gtacttattc caattccact atttcttctg gcactcctgt ggtgttcccg 180
   gatcctactg aggctgcagc gctggtggag gcgattaaca acgctaagtc tgtcactttg 240
   ttctgcggtg cgggcgtgaa gaatgctcgc gcgcaggtgt tggagttggc ggagaagatt 300
   aaatcaccga tcgggcatgc gctgggtggt aagcagtaca tccagcatga gaatccgttt 360
   gaggtcggca tgtctggcct gcttggttac ggcgcctgcg tggatgcgtc caatgaggcg 420
   gatctgctga ttctattggg tacggatttc ccttattctg atttccttcc taaagacaac 480
   gttgcccagg tggatatcaa cggtgcgcac attggtcgac gtaccacggt gaagtatccg 540
   gtgaccggtg atgttgctgc aacaatcgaa aatattttgc ctcatgtgaa ggaaaaaaca 600
   gatcgttcct tccttgatcg gatgctcaag gcacacgagc gtaagttgag ctcggtggta 660
   gagacgtaca cacataacgt cgagaagcat gtgcctattc accctgaata cgttgcctct 720
   attttgaacg agctggcgga taaggatgcg gtgtttactg tggataccgg catgtgcaat 780
   gtgtggcatg cgaggtacat cgagaatccg gagggaacgc gcgactttgt gggttcattc 840
   cgccacggca cgatggctaa tgcgttgcct catgc 875
<210> 7
   <211> 15
   <212> PRT
   <213> Brevibacterium flavum MJ-223
<400> 7
   Met Val Ile Phe Gly Val Thr Gly Asp Leu Ala Arg Lys Lys Leu
   1 5 10 15
<210> 8
   <211> 16
   <212> PRT
   <213> Corynebacterium glutamicum
<400> 8
   Val Ser Thr Asn Thr Thr Pro Ser Ser Trp Thr Asn Pro Leu Arg Asp
   1 5 10 15
<210> 9
   <211> 15
   <212> PRT
   <213> Corynebacterium glutamicum
<400> 9
   Ser Thr Asn Thr Thr Pro Ser Ser Trp Thr Asn Pro Leu Arg Asp
   1 5 10 15
<210> 10
   <211> 20
   <212> DNA
   <213> Artificial Sequence
<220>
   <223> Primer zwf-forward
<400> 10
   tcgacgcggt tctggagcag 20
<210> 11
   <211> 21
   <212> DNA
   <213> Artificial Sequence
<220>
   <223> zwf-reverse
<400> 11
   ctaaattatg gcctgcgcca g 21